(19) Europäisches Patentamt
European Patent Office
Office européen des brevets

(11) Veröffentlichungsnummer: **0 042 100**
**A1**

(12) # EUROPÄISCHE PATENTANMELDUNG

(21) Anmeldenummer: **81104239.9**

(22) Anmeldetag: **03.06.81**

(51) Int. Cl.³: **C 07 D 487/04**, C 07 D 237/04, A 61 K 31/50 // (C07D487/04, 237/00, 231/00)

(30) Priorität: **13.06.80 GB 8019342**
**11.03.81 GB 8107621**

(43) Veröffentlichungstag der Anmeldung: **23.12.81 Patentblatt 81/51**

(84) Benannte Vertragsstaaten: **AT BE CH DE FR GB IT LI LU NL SE**

(71) Anmelder: **F. HOFFMANN-LA ROCHE & CO. Aktiengesellschaft, CH-4002 Basel (CH)**

(72) Erfinder: **Hassall, Cedric Herbert, 6 Ashcroft Close, Harpenden, Herts (GB)**
Erfinder: **Lawton, Geoffrey, 7 Kardwell Close, Hitchin, Herts (GB)**
Erfinder: **Moody, Christopher John, 3 Damask Close Weston, Stevenage, Herts (GB)**

(74) Vertreter: **Lederer, Franz, Dr. et al, Patentanwälte Dr. Lederer Franz Meyer-Roxlau Reiner F. Lucile-Grahn-Strasse 22, D-8000 München 80 (DE)**

(54) **Pyrazolopyridazin-Derivate, Zwischenprodukte und Verfahren zu deren Herstellung, sowie diese enthaltende Arzneimittel.**

(57) Die vorliegende Erfindung betrifft Pyrazolopyridazin-Derivate der allgemeinen Formel

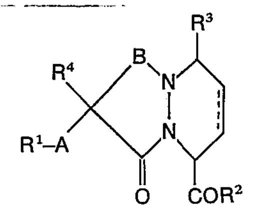

I

worin A gegebenenfalls durch niederes Alkyl substituiertes Methylen, Aethylen oder Propylen, B eine Carbonyl- oder Methylengruppe, $R^1$ Halogen, Carboxyl, niederes Alkoxycarbonyl, Hydroxyaminocarbonyl, Mercapto, niederes Alkanoylthio oder Aryl-niederes Alkylthio, $R^2$ Hydroxy, niederes Alkoxy oder Amino und $R^3$ Wasserstoff, niederes Alkyl, Aryl oder Aryl-niederes Alkyl bedeuten, $R^4$ Wasserstoff, niederes Alkyl, Aryl, Aryl-niederes Alkyl oder die Gruppe $-A-R^1$ bedeutet, wobei A und $R^1$ obige Bedeutung besitzen, und die gestrichelte Linie eine fakultative Bindung bedeutet, wenn B eine Carbonylgruppe bedeutet,
und Salze von Carbonsäuren der allgemeinen Formel I mit pharmazeutisch annahmbaren Basen, Verfahren zu deren Herstellung und diese enthaltende Arzneimittel. Diese Verbindungen und Salze sind nützlich als antihypertensive Mittel.

EP 0 042 100 A1

0042100

F.Hoffmann-La Roche & Co.Aktiengesellschaft, Basel/Schweiz

2. Juni 1981

RAN 4019/85

# Pyrazolopyridazin-Derivate, Zwischenprodukte und Verfahren zu deren Herstellung, sowie diese enthaltende Arzneimittel.

Die vorliegende Erfindung betrifft Pyrazolopyridazin-Derivate. Im speziellen betrifft sie Pyrazolopyridazin-Derivate, ein Verfahren zur Herstellung von solchen Verbindungen und Arzneimittel enthalten solche Verbindungen. Die vorliegende Erfindung betrifft ebenfalls die Verwendung dieser Verbindungen.

Die erfindungsgemässen Pyrazolopyridazin-Derivate sind Verbindungen der allgemeinen Formel

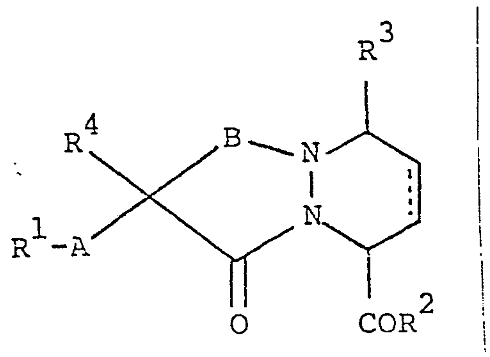

I

worin A gegebenenfalls durch niederes Alkyl substituiertes Methylen, Aethylen oder Propylen, B eine Carbonyl- oder Methylengruppe, $R^1$ Halogen, Carboxyl, niederes Alkoxycarbonyl, Hydroxyaminocarbonyl, Mercapto, niederes Alkanoylthio oder Aryl-niederes Alkylthio,

Nt/21.5.1981

COMPLETE DOCUMENT

$R^2$ Hydroxy, niederes Alkoxy oder Amino und $R^3$ Wasserstoff, niederes Alkyl, Aryl oder Aryl-niederes Alkyl bedeuten, $R^4$ Wasserstoff, niederes Alkyl, Aryl, Aryl-niederes Alkyl oder die Gruppe -A-$R^1$ bedeutet, wobei A und $R^1$ obige Bedeutung besitzen, und die gestrichelte Linie eine fakultative Bindung bedeutet, wenn B eine Carbonylgruppe bedeutet,

und Salze von Verbindungen der allgemeinen Formel I, worin $R^1$ Carboxy und/oder $R^2$ Hydroxy bedeuten, mit pharmazeutisch annehmbaren Basen.

Der in der vorliegenden Beschreibung verwendete Ausdruck "niederes Alkyl", für sich allein genommen oder in Kombinationen, bedeutet geradkettige oder verzweigte Alkylreste mit 1 bis 6 Kohlenstoffatomen, wie Methyl, Aethyl, Propyl, Isopropyl, Butyl, t-Butyl, Pentyl und Hexyl. Der Ausdruck "niederes Alkoxy", für sich allein genommen oder in Kombinationen, bedeutet geradkettige oder verzweigte Alkoxygruppen mit 1 bis 6 Kohlenstoffatomen, wie Methoxy, Aethoxy, Propoxy, Isopropoxy und dergleichen. Beispiele für niederes Alkoxycarbonyl sind Methoxycarbonyl, Aethoxycarbonyl, etc. Der niedere Alkanoylrest einer niederen Alkanoylthiogruppe leitet sich von einer geradkettigen oder verzweigten Fettsäure ab, welche vorzugsweise bis zu 6 Kohlenstoffatomen enthält, wie Essigsäure, Propionsäure, Buttersäure,Isobuttersäure, Valeriansäure, Pivalinsäure und dergleichen; beispielhaft für niedere Alkanoylthiogruppen sind demnach Acetylthio, Propionylthio und dergleichen. Der Ausdruck "Aryl", für sich allein genommen oder in Kombinationen, bedeutet gegebenenfalls durch Halogen, niederes Alkyl, niederes Alkoxy, Trifluormethyl oder dergleichen substituiertes Phenyl. Der Ausdruck "Aryl-niederes alkylthio" umfasst Reste wie Benzylthio. Der Ausdruck "Halogen" bedeutet Fluor, Chlor, Brom oder Jod.

In einer bevorzugten Ausführungsform umfasst die vorliegende Erfindung Verbindungen der allgemeinen Formel I, worin B eine Carbonylgruppe bedeutet. $R^1$ bedeutet vorzugs-

weise Mercapto. $R^2$ bedeutet vorzugsweise Hydroxy. Die bevorzugte Bedeutungsmöglichkeit von $R^3$ ist Wasserstoff. $R^4$ bedeutet vorzugsweise Wasserstoff, niederes Alkyl, insbesondere Methyl oder Aethyl, oder Aryl-niederes Alkyl, insbesondere Benzyl.

Aus dem obigen folgt, dass Verbindungen der allgemeinen Formel I, worin B eine Carbonylgruppe, $R^1$ Mercapto, $R^2$ Hydroxy und $R^4$ Wasserstoff, Methyl, Aethyl oder Benzyl bedeuten, besonders bevorzugt sind.

Eine ganz besonders bevorzugte Verbindung der allgemeinen Formel I ist:

2,3,5,8-Tetrahydro-2-(2-mercaptoäthyl)-2-methyl-1,3-dioxo-1H-pyrazolo[1,2-a]pyridazin-5-carbonsäure.

Weitere bevorzugte Verbindungen der allgemeinen Formel I sind:

Hexahydro-2-mercaptomethyl-2-methyl-1,3-dioxo-1H-pyrazolo[1,2-a]pyridazin-5-carbonsäure,

Hexahydro-2-(2-mercaptoäthyl)-2-methyl-1,3-dioxo-1H-pyrazolo[1,2-a]pyridazin-5-carbonsäure,

Hexahydro-2-(2-mercaptoäthyl)-1,3-dioxo-1H-pyrazolo-[1,2-a]pyridazin-5-carbonsäure,

Hexahydro-2,2-bis(2-mercaptoäthyl)-1,3-dioxo-1H-pyrazolo[1,2-a]pyridazin-5-carbonsäure,

2-Aethyl-hexahydro-2-(2-mercaptoäthyl)-1,3-dioxo-1H-pyrazolo[1,2-a]pyridazin-5-carbonsäure,

2-Benzyl-2,3,5,8-tetrahydro-2-(2-mercaptoäthyl)-1,3-dioxo-1H-pyrazolo[1,2-a]pyridazin-5-carbonsäure,

2-Aethyl-2,3,5,8-tetrahydro-2-(2-mercaptoäthyl)-1,3-dioxo-1H-pyrazolo[1,2-a]pyridazin-5-carbonsäure,

Hexahydro-2-mercaptomethyl-3-oxo-1H-pyrazolo[1,2-a]-pyridazin-5-carbonsäure und

Hexahydro-2-(2-mercaptoäthyl)-2-methyl-3-oxo-1H-pyrazolo[1,2-a]pyridazin-5-carbonsäure.

Weitere interessante Verbindungen, welche von der allgemeinen Formel I umfasst werden, sind:

Diäthyl-5-t-butoxycarbonyl-hexahydro-1,3-dioxo-1H-pyrazolo[1,2-a]pyridazin-2,2-diacetate,

2,2-Bis(2-äthoxycarbonyläthyl)-hexahydro-1,3-dioxo-1H-pyrazolo[1,2-a]pyridazin-5-carboxylat,

t-Butyl-2,2-bis(acetylthiomethyl)-hexahydro-1,3-dioxo-1H-pyrazolo[1,2-a]pyridazin-5-carboxylat,

t-Butyl-2,2-bis(2-acetylthioäthyl)-hexahydro-1,3-dioxo-1H-pyrazolo[1,2-a]pyridazin-5-carboxylat,

t-Butyl-2-(2-acetylthioäthyl)-hexahydro-1,3-dioxo-1H-pyrazolo[1,2-a]pyridazin-5-carboxylat,

t-Butyl-2-acetylthiomethyl-hexahydro-2-methyl-1,3-dioxo-1H-pyrazolo[1,2-a]pyridazin-5-carboxylat,

t-Butyl-2-(2-acetylthioäthyl)-hexahydro-2-methyl-1,3-dioxo-1H-pyrazolo[1,2-a]pyridazin-5-carboxylat,

t-Butyl-2-(3-acetylthiopropyl)-hexahydro-2-methyl-1,3-dioxo-1H-pyrazolo[1,2-a]pyridazin-5-carboxylat,

Diäthyl-5-carboxy-hexahydro-1,3-dioxo-1H-pyrazolo-[1,2-a]pyridazin-2,2-diacetat,

5-Carboxy-hexahydro-1,3-dioxo-1H-pyrazolo[1,2-a]-pyridazin-2,2-diacetat,

2-(2-Acetylthioäthyl)-hexahydro-2-methyl-1,3-dioxo-1H-pyrazolo[1,2-a]pyridazin-5-carbonsäure,

2-(3-Acetylthiopropyl)-hexahydro-2-methyl-1,3-dioxo-1H-pyrazolo[1,2-a]pyridazin-5-carbonsäure,

Methyl-2,3,5,8-tetrahydro-2-(2-jodäthyl)-2-methyl-1,3-dioxo-1H-pyrazolo[1,2-a]pyridazin-5-carboxylat,

Methyl-2-(2-acetylthioäthyl)-2,3,5,8-tetrahydro-2-methyl-1,3-dioxo-1H-pyrazolo[1,2-a]pyridazin-5-carboxylat,

t-Butyl-2-(2-acetylthioäthyl)-2-äthyl-hexahydro-1,3-dioxo-1H-pyrazolo[1,2-a]pyridazin-5-carboxylat,

2-(2-Acetylthioäthyl)-2-äthyl-hexahydro-1,3-dioxo-1H-pyrazolo[1,2-a]pyridazin-5-carbonsäure,

Methyl-2-benzyl-2,3,5,8-tetrahydro-2-(2-jodäthyl)-1,3-dioxo-1H-pyrazolo[1,2-a]pyridazin-5-carboxylat,

Methyl-2-(2-acetylthioäthyl)-2-benzyl-2,3,5,8-tetrahydro-1,3-dioxo-1H-pyrazolo[1,2-a]pyridazin-5-carboxylat,

Methyl-2-äthyl-2,3,5,8-tetrahydro-2-(2-jodäthyl)-1,3-dioxo-1H-pyrazolo[1,2-a]pyridazin-5-carboxylat,

Methyl-2-(2-acetylthioäthyl)-2-äthyl-2,3,5,8-tetrahydro-1,3-dioxo-1H-pyrazolo[1,2-a]pyridazin-5-carboxylat,

t-Butyl-hexahydro-2-[(N-hydroxycarbamoyl)methyl]-2-methyl-1,3-dioxo-1H-pyrazolo[1,2-a]pyridazin-5-carboxylat,

Hexahydro-2-[(N-hydroxycarbamoyl)methyl]-2-methyl-1,3-dioxo-1H-pyrazolo[1,2-a]pyridazin-5-carbonsäure,

t-Butyl-2-brommethyl-hexahydro-3-oxo-1H-pyrazolo[1,2-a]pyridazin-5-carboxylat,

t-Butyl-2-acetylthiomethyl-hexahydro-3-oxo-1H-pyrazolo[1,2-a]pyridazin-5-carboxylat,

2-Acetylthiomethyl-hexahydro-3-oxo-1H-pyrazolo[1,2-a]-pyridazin-5-carbonsäure und

t-Butyl-hexahydro-2-(2-acetylthioäthyl)-2-methyl-3-oxo-1H-pyrazolo[1,2-a]pyridazin-5-carboxylat.

Die Pyrazolopyridazin-Derivate der allgemeinen Formel I und Salze von Verbindungen der allgemeinen Formel I, worin $R^1$ Carboxy und/oder $R^2$ Hydroxy bedeuten, mit pharmazeutisch annehmbaren Basen können erfindungsgemäss dadurch hergestellt werden, dass man

a) zur Herstellung einer Verbindung der allgemeinen Formel I, worin B eine Carbonylgruppe, $R^1$ niederes Alkoxycarbonyl oder niederes Alkanoylthio und $R^2$ niederes Alkoxy bedeuten und in 6,7-Stellung eine Einfachbindung vorhanden ist, eine Verbindung der allgemeinen Formel

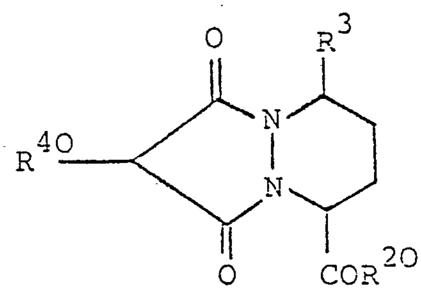

II

worin $R^3$ obige Bedeutung besitzt, $R^{20}$ niederes Alkoxy und $R^{40}$ Wasserstoff, niederes Alkyl, Aryl oder Aryl-niederes Alkyl bedeuten,

mit einer Verbindung der allgemeinen Formel

$$R^{11}-A-X \qquad III$$

worin A obige Bedeutung besitzt, $R^{11}$ niederes Alkoxycarbonyl oder niederes Alkanoylthio und X eine Abgangsgruppe bedeuten,

umsetzt, oder

b) zur Herstellung einer Verbindung der allgemeinen Formel I, worin $R^1$ Halogen bedeutet, eine Verbindung der allgemeinen Formel

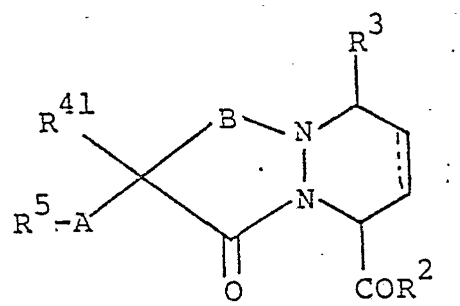

IV

worin A, B, $R^2$, $R^3$ und die gestrichelte Linie obige Bedeutung besitzen, $R^5$ Hydroxy und $R^{41}$ Wasserstoff, niederes Alkyl, Aryl, Aryl-niederes Alkyl oder die Gruppe $-A-R^5$ bedeuten,

entsprechend halogeniert, oder

c) zur Herstellung einer Verbindung der allgemeinen Formel I, worin B eine Methylengruppe, $R^1$ Halogen und $R^2$ niederes Alkoxy bedeuten, eine Verbindung der allgemeinen Formel

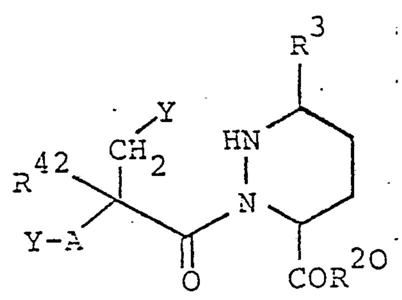

V

worin A, $R^3$ und $R^{20}$ obige Bedeutung besitzen, Y Halogen und $R^{42}$ Wasserstoff, niederes Alkyl, Aryl, Aryl-niederes Alkyl oder die Gruppe -A-Y bedeuten,

cyclisiert, oder

d) zur Herstellung einer Verbindung der allgemeinen Formel I, worin $R^1$ niederes Alkoxycarbonyl bedeutet, in einer entsprechenden Verbindung der allgemeinen Formel I, worin $R^1$ Halogen bedeutet, das Halogenatom in an sich bekannter Weise durch niederes Alkoxycarbonyl ersetzt, oder

e) zur Herstellung einer Verbindung der allgemeinen Formel I, worin $R^1$ niederes Alkanoylthio oder Aryl-niederes Alkylthio bedeutet, eine entsprechende Verbindung der allgemeinen Formel I, worin $R^1$ Halogen bedeutet, oder eine Verbindung der allgemeinen Formel

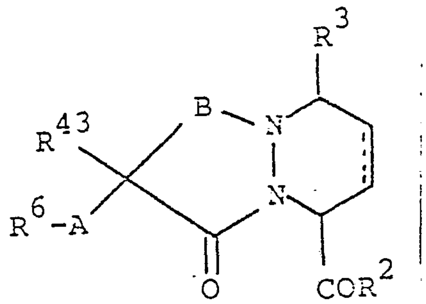

VI

worin A, B, $R^2$, $R^3$ und die gestrichelte Linie obige Bedeutung besitzen, $R^6$ niederes Alkylsulfonyloxy oder Arylsulfonyloxy und $R^{43}$ Wasserstoff, niederes Alkyl, Aryl, Aryl-niederes Alkyl oder die Gruppe $-A-R^6$ bedeuten,

mit einer Verbindung der allgemeinen Formel

$R^7-SH$ VII

worin $R^7$ niederes Alkanoyl oder Aryl-niederes Alkyl bedeutet,

umsetzt, oder

f) zur Herstellung einer Verbindung der allgemeinen Formel I, worin $R^1$ Mercapto bedeutet, in einer entsprechenden Verbindung der allgemeinen Formel I, worin $R^1$ niederes Alkanoylthio oder Aryl-niederes Alkylthio bedeutet, die niedere Alkanoyl- oder Aryl-niedere Alkylgruppe abspaltet, oder

g) zur Herstellung einer Verbindung der allgemeinen Formel I, worin B eine Carbonylgruppe, $R^1$ Halogen, Carboxyl, niederes Alkoxycarbonyl oder Hydroxyaminocarbonyl bedeuten und in 6,7-Stellung eine Einfachbindung vorhanden ist, eine entsprechende Verbindung der allgemeinen Formel I, worin in 6,7-Stellung eine Doppelbindung vorhanden ist, katalytisch hydriert, oder

h) zur Herstellung einer Verbindung der allgemeinen Formel I, worin $R^1$ Hydroxyaminocarbonyl bedeutet, eine entsprechende Verbindung der allgemeinen Formel I, worin $R^1$ niederes Alkoxycarbonyl bedeutet, oder eine Verbindung der allgemeinen Formel

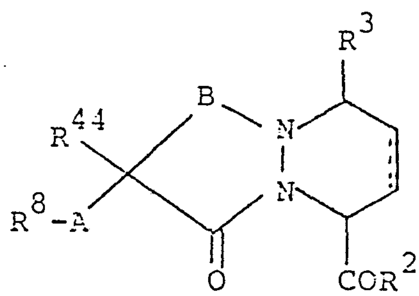

VIII

worin A, B, $R^2$, $R^3$ und die gestrichelte Linie obige Bedeutung besitzen, $R^8$ Aryloxycarbonyl und $R^{44}$ Wasserstoff, niederes Alkyl, Aryl, Aryl-niederes Alkyl oder die Gruppe $-A-R^8$ bedeuten,

mit Hydroxylamin umsetzt, oder

i) zur Herstellung einer Verbindung der allgemeinen Formel I, worin $R^1$ niederes Alkoxycarbonyl und/oder $R^2$ niederes Alkoxy bedeuten, eine entsprechende Verbindung der allgemeinen Formel I, worin $R^1$ Carboxy und/oder $R^2$

Hydroxy bedeuten, verestert, oder

j) zur Herstellung einer Verbindung der allgemeinen Formel I, worin $R^2$ Amino bedeutet, eine entsprechende Verbindung der allgemeinen Formel I, worin $R^2$ Hydroxy bedeutet, amidiert, oder

k) zur Herstellung einer Verbindung der allgemeinen Formel I, worin $R^1$ Carboxy und/oder $R^2$ Hydroxy bedeuten, eine entsprechende Verbindung der allgemeinen Formel I, worin $R^1$ niederes Alkoxycarbonyl und/oder $R^2$ niederes Alkoxy bedeuten, mit einer Säure oder mit einer Base behandelt, oder

l) zur Herstellung einer Verbindung der allgemeinen Formel I, worin B eine Carbonylgruppe, $R^1$ Carboxyl und $R^2$ niederes Alkoxy bedeuten und in 6,7-Stellung eine Einfachbindung vorhanden ist, eine Verbindung der allgemeinen Formel

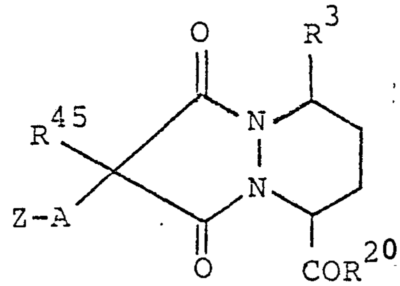

IX

worin A, $R^3$ und $R^{20}$ obige Bedeutung besitzen, Z Benzyloxycarbonyl und $R^{45}$ Wasserstoff, niederes Alkyl, Aryl, Aryl-niederes Alkyl oder die Gruppe -A-Z bedeuten,

debenzyliert, oder

m) zur Herstellung einer Verbindung der allgemeinen Formel I, worin $R^1$ Carboxy bedeutet, eine Verbindung der obigen allgemeinen Formel IV oxydiert, und

n) erwünschtenfalls ein erhaltenes Gemisch von Diastereoisomeren in die entsprechenden Racemate auftrennt, und/oder

o) erwünschtenfalls ein erhaltenes Racemat in die entsprechenden Antipoden spaltet und/oder

p) erwünschtenfalls eine erhaltene Verbindung der allgemeinen Formel I, worin $R^1$ Carboxy und/oder $R^2$ Hydroxy bedeuten, mit einer pharmazeutisch annehmbaren Base in ein Salz überführt.

Die mit X bezeichnete Abgangsgruppe in einer Verbindung der allgemeinen Formel III kann eine herkömmliche Abgangsgruppe sein; beispielsweise ein Chlor-, Brom- oder Jodatom oder eine niedere Alkylsulfonyloxygruppe (z.B. Methansulfonyloxy) oder eine Arylsulfonyloxygruppe (z.B. p-Toluolsulfonyloxy). X bedeutet vorzugsweise eine Bromatom.

Die Reaktion einer Verbindung der allgemeinen Formel II mit einer Verbindung der allgemeinen Formel III, gemäss Verfahrensvariante a), wird zweckmässigerweise in einem inerten organischen Lösungsmittel in Gegenwart einer Base durchgeführt. Die Reaktion kann beispielsweise mit einem Alkalimetall-niederen Alkoxid im entsprechenden niederen Alkohol, z.B. mit Natriumäthoxid in Aethanol, oder mit einem Alkalimetallhydrid, wie Natriumhydrid, in Dimethylformamid durchgeführt werden. Die Reaktionstemperatur ist dabei nicht kritisch; verwendet man als Base ein Alkalimetall-niederen Alkoxid, so wird die Reaktion zweckmässigerweise bei etwa Raumtemperatur durchgeführt, verwendet man als Base ein Alkalimetallhydrid, so wird die Reaktion zweckmässigerweise bei erhöhter Temperatur durchgeführt.

Verwendet man als Ausgangsstoff eine Verbindung der allgemeinen Formel II, worin $R^{40}$ Wasserstoff bedeutet, so kann man unter gewissen Umständen ein Gemisch von 2-monosubstituierter und 2,2-disubstituierter Verbindung der

allgemeinen Formel I erhalten, das leicht aufgetrennt werden kann.

Die Halogenierung einer Verbindung der allgemeinen Formel IV gemäss Verfahrensvariante b) kann in an sich bekannter Weise durchgeführt werden. Die Halogenierung kann beispielsweise so durchgeführt werden, dass man eine Verbindung der allgemeinen Formel IV mit einem Halogenierungsmittel, wie Phosphortrichlorid, Phosphorpentachlorid, Phosphortribromid, Thionylchlorid, etc., behandelt. Diese Halogenierung wird zweckmässigerweise in einem Aether (z.B. Diäthyläther, etc.) und in einem Temperaturbereich von etwa -10°C bis +30°C, vorzugsweise bei etwa Raumtemperatur, durchgeführt. In einer bevorzugte Ausführungsform behandelt man eine Verbindung der allgemeinen Formel IV zuerst mit einem Sulfonylierungsmittel (z.B. mit einem niederen Alkansulfonylhalogenid, wie Methansulfonylchlorid, oder mit einem Arylsulfonylhalogenid, wie p-Toluolsulfonylchlorid), zweckmässigerweise in Gegenwart einer tertiären organischen Base, wie Pyridin, und ersetzt die Sulfonyloxygruppe in der erhaltenen Verbindung durch ein Jodatom, und zwar durch Behandeln mit einem Alkalimetalljodid, vorzugsweise Natriumjodid, zweckmässigerweise in einem inerten organischen Lösungsmittel, z.B. in einem di-niederen Alkylketon (z.B. Aceton) und bei erhöhter Temperatur (z.B. bei der Rückflusstemperatur der Reaktionsmischung).

Die Cyclisierung einer Verbindung der allgemeinen Formel V gemäss Verfahrensvariante c) kann in an sich bekannter Weise durchgeführt werden; beispielsweise durch Behandeln einer Verbindung der allgemeinen Formel V mit einer geeigneten organischen Säure, wie Essigsäure. Zweckmässigerweise wird die Cyclisierung in situ durchgeführt, d.h. ohne Isolierung der Verbindung der allgemeinen Formel V aus dem Medium, indem es vorgängig hergestellt worden ist.

Die Verfahrensvariante d) kann in an sich bekannter Weise durchgeführt werden. Beispielsweise kann man eine Verbindung der allgemeinen Formel I, worin $R^1$ Halogen bedeutet, zusammen mit einem Alkalimetallcyanid, insbesondere mit Kaliumcyanid, in einem wässrigen Alkohol, insbesondere in wässrigem Aethanol, erhitzt. Das erhaltene Nitril kann anschliessend mit konzentrierter Schwefelsäure oder konzentrierter Salzsäure im entsprechenden niederen Alkohol erhitzt werden. Man erhält somit die gewünschte Verbindung der allgemeinen Formel I, worin $R^1$ niederes Alkoxycarbonyl bedeutet. Andererseits ist es auch möglich eine Verbindung der allgemeinen Formel I, worin $R^1$ Halogen bedeutet, in eine Verbindung der allgemeinen Formel I, worin $R^1$ niederes Alkoxycarbonyl bedeutet, überzuführen, indem man ein Malonat oder dergleichen in an sich bekannter Weise verwendet.

Die Reaktion einer Verbindung der allgemeinen Formel I, worin $R^1$ Halogen bedeutet, oder eine Verbindung der allgemeinen Formel VI mit einer Verbindung der allgemeinen Formel VII, gemäss Verfahrensvariante e), wird vorzugsweise in Gegenwart einer Base und in einem inerten Lösungsmittel durchgeführt. Geeignete Basen sind z.B. Alkalimetallhydroxide (z.B. Natriumhydroxid und Kaliumhydroxid), Alkalimetallhydride (z.B. Natriumhydrid und Kaliumhydrid), Alkalimetall-niedere Alkoxide (z.B. Natriummethoxid, Natriumäthoxid, etc.) und Alkalimetallcarbonate (z.B. Natriumcarbonat und Kaliumcarbonat). Bedeutet $R^7$ in einer Verbindung der allgemeinen Formel VII niederes Alkanoyl, so sind di-niedere Alkylketone (z.B. Aceton) und Dimethylformamid, oder, wenn ein Alkalimetallcarbonat als Base verwendet wird, eine Mischung aus Wasser und einem chlorierten Kohlenwasserstoff (z.B. Methylenchlorid), oder eine Mischung aus Wasser und Essigester geeignete Lösungsmittel. Bedeutet $R^7$ in einer Verbindung der allgemeinen Formel VII Aryl-niederes Alkyl, so sind Wasser, Dimethylformamid und dergleichen geeignete Lösungsmittel. Es kann zweckmässig sein, eine Verbindung der allgemeinen Formel VII, worin

$R^7$ niederes Alkanoyl bedeutet, in Form eines Alkalimetallsalzes (z.B. eines Kaliumsalzes) zu verwenden, und, wenn $R^1$ in einer Verbindung der allgemeinen Formel I nicht Jod bedeutet, die Reaktion in Gegenwart einer katalytischen Menge eines Alkalimetalljodides (z.B. von Kaliumjodid) durchzuführen. Die Reaktionstemperatur ist nicht kritisch; man kann demnach in einem Bereich von etwa 10°C bis zur Siedetemperatur der Reaktionsmischung arbeiten.

Die Spaltung gemäss Verfahrensvariante f) kann in an sich bekannter Weise durchgeführt werden, wobei natürlich die Art der zu spaltenden Gruppe berücksichtigt werden muss. Soll beispielsweise eine niedere Alkanoylgruppe abgespalten werden, so kann man ein wässriges Alkalimetallhydroxid (z.B. wässriges Natriumhydroxid oder wässriges Kaliumhydroxid), wässriges Ammoniak, einen niederen Alkohol (z.B. Methanol) in Gegenwart des entsprechenden Alkalimetall-niederen Alkoxides (z.B. Natriummethoxid) oder eine Mineralsäure, wie Salzsäure, verwenden, wobei man vorzugsweise bei erhöhter Temperatur arbeitet. Vorzugsweise verwendet man wässriges Ammoniak. Soll eine Aryl-niedere Alkylgruppe abgespalten werden, so kann man auch Natrium in flüssigem Ammoniak verwenden.

Gemäss Verfahrensvariante g) wird eine Verbindung der allgemeinen Formel I, worin B eine Carbonylgruppe bedeuten und in 6,7-Stellung eine Doppelbindung vorhanden ist, katalytisch hydriert. Geeignete Katalysatoren sind Edelmetallkatalysatoren, wie z.B. Palladium, Platin, Ruthenium, Rhodium und Raney-Nickel. Der Katalysator kann dabei auf einem geeigneten Trägermaterial aufgebracht sein (z.B. Palladium/Kohle, Rhodium/Aluminiumoxid und dergleichen). Die katalytische Hydrierung kann in einem herkömmlichen inerten organischen Lösungsmittel, wie z.B. einem aromatischen Kohlenwasserstoff (z.B. Benzol, Toluol, Xylol, etc.), einem niederen Alkohol (z.B. Methanol, Aethanol, etc.) oder in einem Aether (z.B. Dioxan, etc.) durchgeführt werden. Die katalytische Hydrierung wird vorteilhafterweise

bei Raumtemperatur und Atmosphärendruck durchgeführt.

Gemäss Verfahrensvariante h) wird eine Verbindung der allgemeinen Formel I, worin $R^1$ niederes Alkoxycarbonyl bedeutet, oder eine Verbindung der allgemeinen Formel VIII mit Hydroxylamin umgesetzt. Die Reaktion wird zweckmässigerweise in einem inerten organischen Lösungsmittel durchgeführt; z.B. in einem niederen Alkohol, insbesondere in Methanol. Obwohl die Reaktion in einem Temperaturbereich von etwa 0°C bis zur Siedetemperatur der Reaktionsmischung durchgeführt werden kann, arbeitet man vorzugsweise bei etwa Raumtemperatur. Das Hydroxylamin kann als Säureadditionssalz (z.B. als Hydrochlorid) eingesetzt werden, wobei in diesem Fall eine geeignete Base (z.B. ein Alkalimetallhydroxid, insbesondere Kaliumhydroxid) der Reaktionsmischung zugegeben wird.

Die Veresterung einer Verbindung der allgemeinen Formel I, gemäss Verfahrensvariante i), kann in an sich bekannter Weise durchgeführt werden. Man kann beispielsweise eine Verbindung der allgemeinen Formel I, worin $R^1$ Carboxy und/oder $R^2$ Hydroxy bedeuten, in Gegenwart einer geeigneten Säure (z.B. einer Mineralsäure, wie Salzsäure) mit einem niederen Alkohol (z.B. mit Methanol, Aethanol, etc.), oder mit einem geeigneten Diazoalkan (z.B. mit Diazomethan) umsetzen. Es ist aber auch möglich, eine Verbindung der allgemeinen Formel I, worin $R^1$ Carboxy und/oder $R^2$ Hydroxy bedeuten, in an sich bekannter Weise (z.B. durch Behandeln mit einem Chlorierungsmittel, wie Thionylchlorid, Phosphortrichlorid oder Phosphorpentachlorid) in das entsprechende Säurechlorid überzuführen und dieses, ebenfalls in an sich bekannter Weise, mit einem niederen Alkohol umsetzen. Einen t-Butylester kann man auch erhalten, indem man eine Verbindung der allgemeinen Formel I, worin $R^1$ Carboxy und/oder $R^2$ Hydroxy bedeuten, in Gegenwart von Schwefelsäure mit Isobuten umsetzt.

Die Amidierung, gemäss Verfahrensvariante j), kann in an sich bekannter Weise durchgeführt werden. Beispielsweise kann man eine Verbindung der allgemeinen Formel I, worin $R^2$ Carboxy bedeutet, wie oben beschrieben in das entsprechende Säurechlorid überführen und dieses in an sich bekannter Weise mit Ammoniak behandeln; man erhält dabei eine Verbindung der allgemeinen Formel I, worin $R^2$ Amino bedeutet.

Gemäss Verfahrensvariante k) wird eine Verbindung der allgemeinen Formel I, worin $R^1$ niederes Alkoxycarbonyl und/oder $R^2$ niederes Alkoxy bedeuten, in eine Verbindung der allgemeinen Formel I, worin $R^1$ Carboxy und/oder $R^2$ Hydroxy bedeuten, übergeführt. Man kann diese Reaktion in an sich bekannter Weise durchführen; beispielsweise durch Behandeln des Ausgangsmaterials mit einem wässrigen Alkalimetallhydroxid, wie Natriumhydroxid oder Kaliumhydroxid, oder mit einer wässrigen Mineralsäure, wie Salzsäure, zweckmässigerweise in einem Temperaturbereich von etwa Raumtemperatur bis zum Siedepunkt der Reaktionsmischung oder, wenn $R^1$ t-Butoxycarbonyl und/oder $R^2$ t-Butoxy bedeuten, durch Behandeln mit wasserfreier Säure.

Die Debenzylierung, gemäss Verfahrensvariante l), kann in an sich bekannter Weise durchgeführt werden; beispielsweise unter Verwendung von elementarem Wasserstoff in Gegenwart eines Katalysators (z.B. Palladium/Kohle) in einem niederen Alkohol (z.B. in Methanol) bei Raumtemperatur und Atmosphärendruck.

Die Oxidation einer Verbindung der allgemeinen Formel IV, gemäss Verfahrensvariante m), kann in Analogie zu den an sich bekannten Verfahren zur Oxidation von Alkoholen zu den entsprechenden Carbonsäuren durchgeführt werden. Man kann beispielsweise ein chromhaltiges Oxidationsmittel verwenden.

Die Verbindungen der allgemeinen Formel I enthalten ein asymmetrisches Zentrum (5-Stellung) und können demnach in racemischer oder optisch aktiver Form vorliegen. Verbindungen der allgemeinen Formel I, die mehr als ein asymmetrisches Zentrum enthalten, können in verschiedenen diastereoisomeren Formen vorliegen. Die vorliegende Erfindung umfasst sämtliche möglichen Stereoisomeren von Verbindungen der allgemeinen Formel I und alle möglichen diastereoisomeren Mischungen und Racemate. Die Auftrennung von diastereoisomeren Mischungen in die entsprechenden diastereoisomeren Racemate, gemäss Verfahrensvariante n), und die Spaltung von Racematen in die optischen Antipoden, gemäss Verfahrensvariante o), können in an sich bekannter Weise durchgeführt werden.

Verbindungen der allgemeinen Formel I, worin $R^1$ Carboxy und/oder $R^2$ Hydroxy bedeuten, können, gemäss Verfahrensvariante p), mit pharmazeutisch annehmbaren Basen in Salze übergeführt werden. Man kann diese Verbindungen beispielsweise mit Alkalimetallhydroxiden (z.B. mit Natriumhydroxid und Kaliumhydroxid), Erdalkalimetallhydroxiden (z.B. Calciumhydroxid und Magnesiumhydroxid), organischen Basen (z.B. Dicyclohexylamin, etc.), basischen Aminosäuren (z.B. Lysin und Arginin) und dergleichen behandeln.

Die in Verfahrensvariante a) als Ausgangsstoffe eingesetzten Verbindungen der allgemeinen Formel II sind neu und ebenfalls Gegenstand der vorliegenden Erfindung. Sie können gemäss dem nachfolgenden Formelschema I, worin $R^3$, $R^{20}$, $R^{40}$ und Z obige Bedeutung besitzen und $R^9$ niederes Alkyl bedeutet, hergestellt werden:

Formelschema I

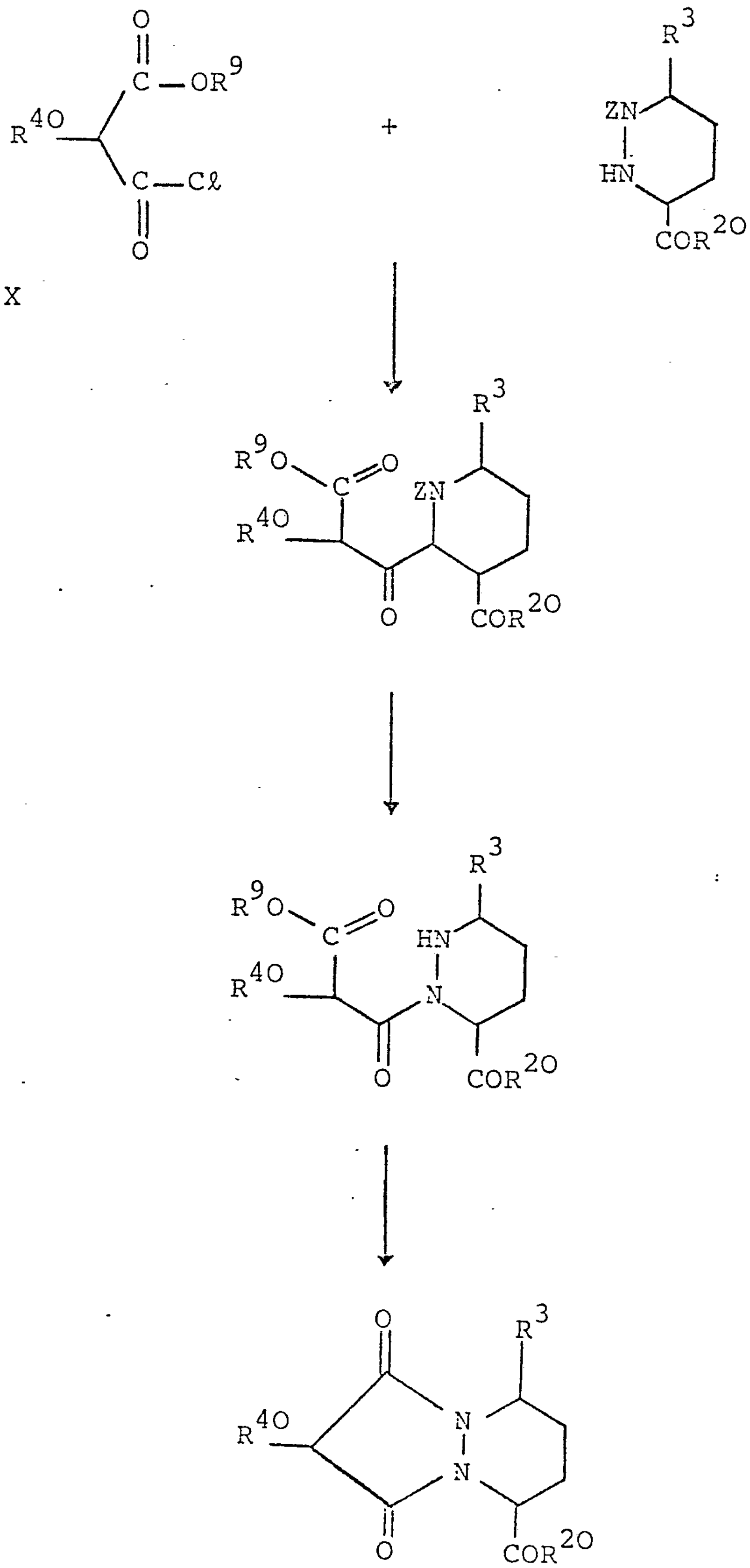

X

XI

XII

XIII

II

Die Verbindungen der allgemeinen Formeln X und XI sind bekannt oder können in Analogie zur Herstellung der bekannten Verbindungen hergestellt werden.

Die Reaktion einer Verbindung der allgemeinen Formel X mit einer Verbindung der allgemeinen Formel XI zu einer Verbindung der allgemeinen Formel XII kann unter den Bedingungen einer Schotten-Baumann-Reaktion durchgeführt werden, und zwar in Gegenwart eines inerten organischen Lösungsmittels (z.B. eines halogenierten Kohlenwasserstoffes, wie Methylenchlorid) und in Gegenwart von verdünnter Natronlauge bei etwa Raumtemperatur.

In einem nächsten Schritt wird die mit Z bezeichnete Benzyloxycarbonylgruppe in einer Verbindung der allgemeinen Formel XII abgespalten. Diese Reaktion wird zweckmässigerweise unter Verwendung von elementarem Wasserstoff in Gegenwart eines Katalysators, wie Palladium/Kohle, bei Raumtemperatur und Atmosphärendruck durchgeführt.

Das gewünschte Ausgangsmaterial der allgemeinen Formel II erhält man schliesslich, indem man eine Verbindung der allgemeinen Formel XIII cyclisiert. Diese Cyclisierung kann man in an sich bekannter Weise durchführen; beispielsweise durch Erhitzen in Gegenwart einer geeigneten organischen Säure, wie Essigsäure.

Die in Verfahrensvariante b) als Ausgangsstoffe eingesetzten Verbindungen der allgemeinen Formel IV sind neu und ebenfalls Gegenstand der vorliegenden Erfindung.

Die Ausgangsstoffe der allgemeinen Formel IV, worin B eine Carbonylgruppe bedeutet, können gemäss dem nachfolgenden Formelschema II, worin A, $R^2$, $R^3$, $R^5$, $R^9$ und $R^{41}$ obige Bedeutung besitzen und $R^{10}$ eine Hydroxygruppe bedeutet, die als leicht spaltbarer Aether geschützt ist (z.B. als Benzyläther, Tetrahydropyranyläther, etc.), hergestellt werden:

Formelschema II

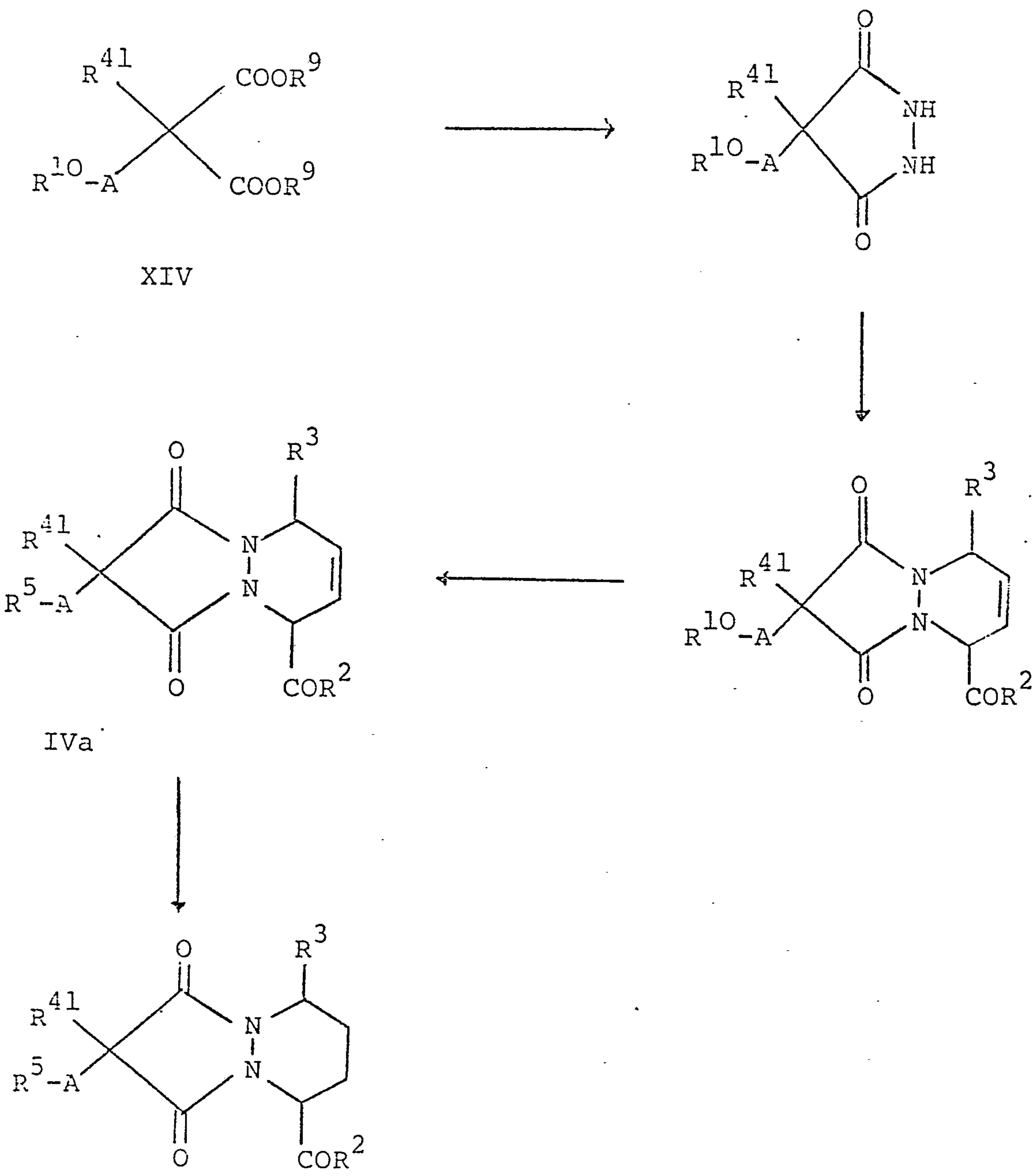

XIV

XV

XVI

IVa

IVb

In einem ersten Schritt wird ein Diester der allgemeinen Formel XIV (eine bekannte Verbindung oder das Analogon einer bekannten Verbindung) mit Hydrazin zu einem Pyrazolidindion-Derivat der allgemeinen Formel XV umgesetzt. Zweckmässigerweise erhitzt man dabei eine Mischung aus dem Diester und wasserfreiem Hydrazin bei erhöhter Temperatur (z.B. bei der Siedetemperatur der Reaktionsmischung).

In einem nächsten Schritt wird das erhaltene Pyrazolidindion-Derivat der allgemeinen Formel XV durch Reaktion mit einer Verbindung der allgemeinen Formel

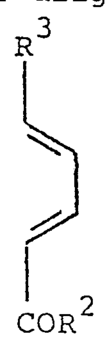

XVII

worin $R^2$ und $R^3$ obige Bedeutung besitzen, unter oxidierenden Bedingungen in eine Verbindung der allgemeinen Formel XVI übergeführt.

Die oxidierenden Bedingungen, welche für die Reaktion eines Pyrazolidindion-Derivates der allgemeinen Formel XV mit einer Verbindung der allgemeinen Formel XVII benötigt werden, erhält man geeigneter Weise dadurch, dass man der Reaktionsmischung ein geeignetes Oxidationsmittel, wie Bleitetraacetat, t-Butyl-hypochlorit oder dergleichen zugibt. Zweckmässigerweise arbeitet man in einem geeigneten inerten Lösungsmittel, beispielsweise in einem aromatischen Kohlenwasserstoff (z.B. Benzol, Toluol, etc.), in einem halogenierten Kohlenwasserstoff (z.B. Methylenchlorid, Chloroform, Chlorbenzol, etc.), in einem di-niederen Alkylketon (z.B. Aceton, Methyläthylketon, etc.), in einem Aether (z.B. Diäthyläther, Dioxan, Tetrahydrofuran, etc.), in Acetonitril, Essigester, etc. Die Reaktionstemperatur ist nicht kritisch, vorzugsweise arbeitet man jedoch bei Raumtemperatur.

Aus einer Verbindung der allgemeinen Formel XVI erhält man einen Ausgangsstoff der allgemeinen Formel IVa dadurch, dass man die Schutzgruppe abspaltet. Diese Spaltung kann man in an sich bekannter Weise durchführen; beispielsweise durch Behandeln mit einer Mineralsäure (z.B. mit einer Halogenwasserstoffsäure, wie Salzsäure) bei etwa Raumtemperatur.

Ein Ausgangsstoff der allgemeinen Formel IVa kann anschliessend in einen Ausgangsstoff der allgemeinen Formel IVb übergeführt werden, und zwar durch katalytische Hydrierung der 6,7-Doppelbindung in Analogie zu Verfahrensvariante g).

Die als Ausgangsstoffe verwendeten Verbindungen der allgemeinen Formel IV, worin B eine Methylengruppe bedeutet, können beispielsweise hergestellt werden, indem man eine Mischung aus einer Verbindung der allgemeinen Formel XI und eine Verbindung der allgemeinen Formel

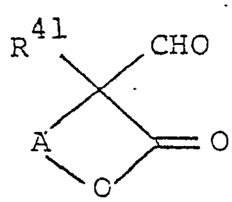

XVIII

worin A und $R^{41}$ obige Bedeutung besitzen, in einem herkömmlichen inerten organischen Lösungsmittel unter neutralen oder sauren Bedingungen katalytisch hydriert. Arbeitet man unter neutralen Bedingungen, so erhält man eine Verbindung der allgemeinen Formel

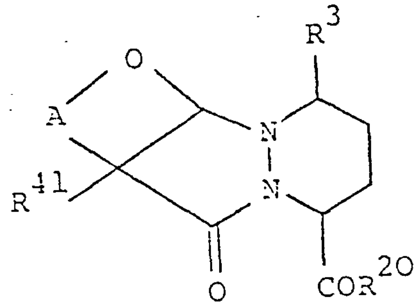

XIX

worin A, $R^3$, $R^{20}$ und $R^{41}$ obige Bedeutung besitzen;

arbeitet man unter sauren Bedingungen, so erhält man eine Verbindung der allgemeinen Formel

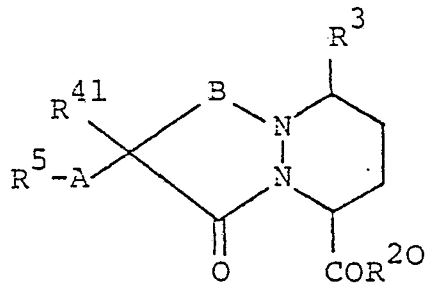

IVc

worin B, $R^3$, $R^5$, $R^{20}$ und $R^{21}$ obige Bedeutung besitzen.

Eine Verbindung der allgemeinen Formel XIX wird durch katalytische Hydrierung unter sauren Bedingungen umgewandelt.

Als Katalysator in der erwähnten katalytischen Hydrierung verwendet man vorzugsweise einen Palladiumkatalysator, wie Palladium/Kohle. Arbeitet man bei der katalytischen Hydrierung unter neutralen Bedingungen, so verwendet man als organisches Lösungsmittel vorzugsweise einen niederen Alkohol, wie Methanol, Aethanol, etc.; arbeitet man unter sauren Bedingungen, so verwendet man als organisches Lösungsmittel vorzugsweise Dioxan oder dergleichen. Die sauren Bedingungen erhält man in herkömmlicher Weise, beispielsweise, indem man der Reaktionsmischung Salzsäure beigibt. Die katalytische Hydrierung wird geeigneter Weise bei Raumtemperatur und Atmosphärendruck durchgeführt.

Erwünschtenfalls kann die mit $R^{20}$ bezeichnete niedere Alkoxygruppe in einer Verbindung der allgemeinen Formel IVc durch Hydroxy oder Amino ersetzt werden, und zwar in an sich bekannter Weise (z.B. wie weiter oben beschrieben).

Die in Verfahrensvariante c) als Ausgangsstoffe verwendeten Verbindungen der allgemeinen Formel V sind neu und ebenfalls Gegenstand der vorliegenden Erfindung. Sie können beispielsweise hergestellt werden, indem man eine

Verbindung der allgemeinen Formel

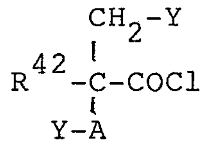

XX

worin A, $R^{42}$ und Y obige Bedeutung besitzen,

mit einer Verbindung der obigen allgemeinen Formel XI umsetzt, und in der erhaltenen Verbindung der allgemeinen Formel

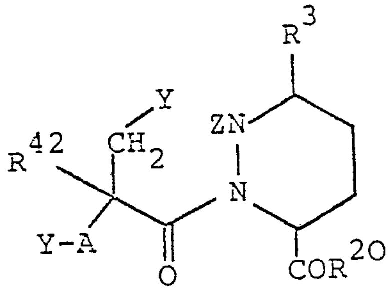

XXI

worin A, $R^3$, $R^{42}$, $R^{20}$, Y und Z obige Bedeutung besitzen,

die Benzyloxycarbonylgruppe abspaltet.

Die Reaktion einer Verbindung der allgemeinen Formel XX (eine bekannte Verbindung oder das Analogon einer bekannten Verbindung) mit einer Verbindung der allgemeinen Formel XI kann in an sich bekannter Weise durchgeführt werden. Beispielsweise kann man in einem inerten organischen Lösungsmittel (z.B. in einem halogenierten Kohlenwasserstoff, wie Methylenchlorid) und in Gegenwart eines säurebindenden Mittels, wie eines Alkalimetallbicarbonates (z.B. Natriumbicarbonat) und bei etwa Raumtemperatur arbeiten.

Die Abspaltung der Benzyloxycarbonylgruppe aus einer Verbindung der allgemeinen Formel XXI kann in Analogie zur weiter oben beschriebenen Reaktion einer Verbindung der allgemeinen Formel XII zu einer Verbindung der allgemeinen Formel XIII durchgeführt werden.

Die in Verfahrensvariante e) als Ausgangsstoffe eingesetzten Verbindungen der allgemeinen Formel VI sind neu und ebenfalls Gegenstand der vorliegenden Erfindung. Sie können beispielsweise dadurch hergestellt werden, dass man eine Verbindung der obigen allgemeinen Formel IV mit einem geeigneten Alkylsulfonyl-halogenid (z.B. Methansulfonylchlorid) oder einem Arylsulfonyl-halogenid (z.B. p-Toluolsulfonylchlorid) in an sich bekannter Weise umsetzt.

Die in Verfahrensvariante h) als Ausgangsstoffe eingesetzten Verbindungen der allgemeinen Formel VIII sind neu und ebenfalls Gegenstand der vorliegenden Erfindung. Sie können beispielsweise in Analogie zur Herstellung von Verbindungen der allgemeinen Formel I, worin $R^1$ niederes Alkoxycarbonyl bedeutet, hergestellt werden; beispielsweise durch Umsetzen einer Verbindung der allgemeinen Formel II mit einer Verbindung der allgemeinen Formel III, worin jedoch $R^{11}$ Aryloxycarbonyl bedeutet.

Die in Verfahrensvariante l) als Ausgangsstoffe eingesetzten Verbindungen der allgemeinen Formel IX sind neu und ebenfalls Gegenstand der vorliegenden Erfindung. Sie können beispielsweise hergestellt werden, indem man eine Verbindung der allgemeinen Formel II mit einer entsprechenden Verbindung der allgemeinen Formel III, worin jedoch $R^{11}$ Benzyloxycarbonyl bedeutet, umsetzt, und zwar in Analogie zur Verfahrensvariante a).

Die erfindungsgemässen Pyrazolopyridazin-Derivate sind nützlich als Arzneimittel, insbesondere als antihypertensive Mittel. Sie hemmen das Angiotensin umwandelnde Enzym (ACE), das die Umwandlung von Angiotensin I zu Angiotensin II zustande bringt, und sind deshalb nützlich, um einen durch Angiotensin bewirkten erhöhten Blutdruck zu vermindern oder zu lindern.

Die Aktivität der vorliegenden Pyrazolopyridazin-Derivate, das das Angiotensin umwandelnde Enzym (ACE) in vitro zu hemmen, kann durch den folgenden Test bestimmt werden.

Die verwendete Methode basiert auf einer Methode von Cushman and Cheung (Biochem. Pharmacol., 20, 1637-1648) unter Berücksichtigung der Modifikationen von Hayakari et al. (Anal. Biochem., 84, 361-369). Das Substrat (Hippuryl-Histidyl-Leucin, 2 mM) wird mit oder ohne Testverbindung (verschiedene Konzentrationen) in Kaliumphosphat-Puffer (pH 8,3; 100 mM), das Natriumchlorid (300 mM) enthält, während 25 Minuten bei 37°C (Gesamtwert 500 µl) mit ACE, das man aus Kaninchenlungen extrahiert hat, inkubiert. Die Reaktion wird durch Zugabe von 3 ml Kaliumphosphat-Puffer (pH 8,3; 200 mM) bei 0°C abgebrochen. Man versetzt anschliessend mit 2,4,6-Trichlor-s-triazin (3%) in 1,5 ml Dioxan und schüttelt die erhaltene Mischung, bis der gelbe Chromophor sich voll entwickelt hat. Die Proben werden anschliessend zentrifugiert, um allenfalls entstandenen Festkörper zu entfernen. Der gelbe Chromophor, der durch Reaktion von 2,4,6-Trichlor-s-triazin mit freier Hippursäure entstanden ist, wird spektrophotometrisch bei 382 nm ausgemessen. Der $IC_{50}$-Wert ist diejenige Konzentration einer Testverbindung, die notwendig ist, um die Spaltung von Hippuryl-Histidyl-Leucin durch ACE unter den vorangehend beschriebenen Bedingungen um 50% zu reduzieren.

Die nachfolgende Tabelle enthält die Resultate, welche mit repräsentativen Vertretern von Verbindungen der allgemeinen Formel I im vorhergehend beschriebenen Test erhalten wurden.

Verbindung A: Hexahydro-2-(2-mercaptoäthyl)-2-methyl-1,3-dioxo-1H-pyrazolo[1,2-a]pyridazin-5-carbonsäure.

Verbindung B: Hexahydro-2-(2-mercaptoäthyl)-2-methyl-3-oxo-1H-pyrazolo[1,2-a]pyridazin-5-carbonsäure.

Verbindung C: 2-Aethyl-2,3,5,8-tetrahydro-2-(2-mercaptoäthyl)-1,3-dioxo-1H-pyrazolo[1,2-a]pyridazin-5-carbonsäure.

Tabelle

| Verbindung | $IC_{50}$ (nanomolar) |
|---|---|
| A | 100 |
| B | 100 |
| C | 43 |

Die erfindungsgemässen Pyrazolopyridazin-Derivate können in Form von pharmazeutischen Präparaten, welche Verbindungen der allgemeinen Formel I zusammen mit geeigneten pharmazeutischen Trägermaterialien enthalten, als Arzneimittel verwendet werden. Als Trägermaterialien kommen dabei für die enterale, beispielsweise orale, oder parenterale Verabreichung geeignete organische oder anorganisch Trägermaterialien in Frage, beispielsweise Wasser, Gelatine, Gummi arabicum, Lactose, Stärke, Magnesiumstearat, Talk, pflänzliche Oele, Polyalkylenglykole, Vaselin und dergleichen. Die pharmazeutischen Präparate können in fester Form, beispielsweise als Tabletten, Dragées, Suppositorien oder Kapseln, oder in flüssiger Form, beispielsweise als Lösungen, Suspensionen oder Emulsionen, verarbeitet werden. Die pharmazeutischen Präparate können üblichen pharmazeutischen Behandlungen, wie Sterilisation, unterworfen werden und/oder Zusatzstoffe enthalten, wie Konservierungsmittel, Stabilisierungsmittel, Netzmittel oder Emulgiermittel, Salze, um den osmotischen Druck zu variieren, oder Puffer. Die pharmazeutischen Präparate

können aber auch andere therapeutisch wertvolle Stoffe enthalten.

Die erfindungsgemässen Pyrazolopyridazin-Derivate können Erwachsenen in einer täglichen Dosis von etwa 0,1 bis 100 mg, vorzugsweise ca. 1 bis 50 mg, pro Kilogramm Körpergewicht verabreicht werden. Die tägliche Dosis kann in einer einzigen Dosis oder in verschiedenen Dosen verabreicht werden. Es sei an dieser Stelle betont, dass die oben angegebenen Dosierungen als Beispiele angegeben wurden, und dass sie, je nach Stärke der zu behandelnden Symptome und Zustand der Patienten, nach oben oder nach unten variieren können und letztlich vom behandelnden Arzt festgelegt werden.

Die nachfolgenden Beispiele dienen der näheren Erläuterung der vorliegenden Erfindung.

Beispiel 1

(A) Man gibt eine Lösung von 13 ml Aethylmalonylchlorid in 500 ml Methylenchlorid und 210 ml 0,5M Natronlauge gleichzeitig und unter Rühren tropfenweise zu einer Lösung von 28,7 g 1-Benzyloxycarbonylpiperazinsäure-t-Butylester in 500 ml Methylenchlorid, rührt die erhaltene Mischung während 4 Stunden bei Raumtemperatur und trennt die verschiedenen Phasen. Durch Chromatographieren der organischen Phase erhält man 25,3 g (65%) 1-Benzyl-3-t-butyl-2-(2-äthoxycarbonylacetyl)hexahydropyridazin-1,3-dicarboxylat vom Schmelzpunkt 45-47°C (aus Diäthyläther/Hexan).

(B) Man hydriert eine Lösung von 25,3 g 1-Benzyl-3-t-butyl-2-(2-äthoxycarbonylacetyl)hexahydropyridazin-1,3-dicarboxylat in 500 ml Methanol über 2,3 g Palladium/Kohle (10%) bei Raumtemperatur und Atmosphärendruck. Man filtriert den Katalysator ab, dampft das Filtrat ein und kristallisiert den Rückstand aus Hexan um. Man erhält 16,9 g (97%) t-Butyl-2-(2-äthoxycarbonylacetyl)hexahydropyridazin-3-carboxylat vom Schmelzpunkt 86-87°C.

(C) Man erhitzt eine Lösung von 16,9 g t-Butyl-2-(2-äthoxycarbonylacetyl)hexahydropyridazin-3-carboxylat in 350 ml Essigsäure während 1,5 Stunden auf 100°C und dampft anschliessend ein. Man chromatographiert das erhaltene Oel an Kieselgel und erhält 9,45 g (66%) t-Butyl-hexahydro-1,3-dioxo-1H-pyrazolo[1,2-a]pyridazin-5-carboxylat als schwach gelben Festkörper vom Schmelzpunkt 121-122°C (aus Chloroform/Hexan).

(D)(a) Man suspendiert 0,4 g Natriumhydrid (80-proz. Dispersion) in 10 ml trockenem Dimethylformamid und versetzt mit einer Lösung von 1,27 g t-Butyl-hexahydro-1,3-dioxo-1H-pyrazolo[1,2-a]pyridazin-5-carboxylat in 10 ml trockenem Dimethylformamid und rührt die erhaltene Mischung bei Raumtemperatur, bis zur Beendigung der Gasentwicklung. Anschliessend versetzt man mit 2,5 g Aethylbromacetat,

BAD ORIGINAL

rührt die Mischung während 16 Stunden bei 20°C, entfernt das Lösungsmittel und verteilt den Rückstand zwischen 2N-Salzsäure und Methylenchlorid. Die organische Phase wird abgetrennt, über Natriumsulfat getrocknet und eingedampft. Durch Chromatographieren des Rückstandes an Kieselgel erhält man 1,49 g (70%) Diäthyl-5-t-butoxycarbonyl-hexahydro-1,3-dioxo-1H-pyrazolo[1,2-a]pyridazin-2,2-diacetat als fabloses Oel.

(D)(b) Man löst 0,43 g Natrium in 20 ml Aethanol, versetzt mit einer Lösung von 2,27 g t-Butyl-hexahydro-1,3-dioxo-1H-pyrazolo[1,2-a]pyridazin-5-carboxylat in 20 ml Aethanol und erhitzt die Mischung während 2 Stunden unter Rückfluss zum Sieden. Anschliessend versetzt man mit 5 g Aethyl-3-brompropionat, erhitzt die Mischung während 2 Stunden unter Rückfluss zum Sieden, entfernt das Lösungsmittel und verteilt den Rückstand zwischen Methylenchlorid und 2N Salzsäure. Die organische Phase wird abgetrennt und eingedampft. Durch Chromatographieren des Rückstandes an Kieselgel erhält man 0,37 g (9%) t-Butyl-2,2-bis(2-äthoxycarbonyläthyl)hexahydro-1,3-dioxo-1H-pyrazolo[1,2-a]-pyridazin-5-carboxylat als Oel.

(D)(c) Man gibt eine Lösung von 1,27 g t-Butyl-hexahydro-1,3-dioxo-1H-pyrazolo[1,2-a]pyridazin-5-carboxylat in 10 ml Dimethylformamid zu einer Suspension von 0,4 g Natriumhydrid in Dimethylformamid und rührt die Mischung bis zu Beendigung der Gasentwicklung bei Raumtemperatur. Man versetzt anschliessend mit 1,86 g S-Brommethylthioacetat, erhitzt die Mischung während 5 Stunden auf 75°C und arbeitet wie unter (D)(a) beschrieben auf. Man erhält 0,15 g (7%) t-Butyl-2,2-bis(acetylthiomethyl)-hexahydro-1,3-dioxo-1H-pyrazolo[1,2-a]pyridazin-5-carboxylat als weissen Festkörper vom Schmelzpunkt 85-86°C (aus Diäthyläther/Hexan).

(D)(d) In Analogie zu den Angaben in Absatz (D)(c) erhält man aus 1,27 g t-Butyl-hexahydro-1,3-dioxo-1H-pyrazolo[1,2-a]pyridazin-5-carbonsäure und 2,4 g S-(2-Bromäthyl)thioacetat 0,09 g (4%) t-Butyl-2,2-bis(2-acetylthioäthyl)-hexahydro-1,3-dioxo-1H-pyrazolo[1,2-a]pyridazin-5-carboxylat als Oel. Man erhält auch 0,075 g (4%) t-Butyl-2-(2-acetylthioäthyl)-hexahydro-1,3-dioxo-1H-pyrazolo-[1,2-a]pyridazin-5-carboxylat als weissen Festkörper vom Schmelzpunkt 86-88°C (aus Diäthyläther).

## Beispiel 2

(A) In Analogie zu den Angaben in Beispiel 1 (A) erhält man aus 1-Benzyloxycarbonylpiperazinsäure-t-Butylester und Aethylmethylmalonylchlorid 36% 1-Benzyl-3-t-butyl-2-(2-äthoxycarbonyl-2-methylacetyl)hexahydropyridazin-1,3-dicarboxylat als Oel.

(B) In Analogie zu den Angaben in Beispiel 1 (B) erhält man aus 3,9 g 1-Benzyl-3-t-butyl-2-(2-äthoxycarbonyl-2-methylacetyl)hexahydropyridazin-1,3-dicarboxylat 2,6 g (95%) t-Butyl-2-(2-äthoxycarbonyl-2-methyl-acetyl)hexahydropyridazin-3-carboxylat als Oel.

(C) In Analogie zu den Angaben in Beispiel 1 (C) erhält man aus 2,6 g t-Butyl-2-(2-äthoxycarbonyl-2-methylacetyl)-hexahydropyridazin-3-carboxylat 1,39 g (63%) t-Butyl-hexahydro-2-methyl-1,3-dioxo-1H-pyrazolo[1,2-a]pyridazin-5-carboxylat als weissen Festkörper vom Schmelzpunkt 127-128°C (aus Chloroform/Hexan).

(D)(a) In Analogie zu den Angaben in Beispiel 1 (D)(c) erhält man aus 7,08 g t-Butyl-hexahydro-2-methyl-1,3-dioxo-1H-pyrazolo[1,2-a]pyridazin-5-carboxylat und 4,98 g S-Brommethylthioacetat 5,59 g (59%) t-Butyl-2-acetylthiomethyl-hexahydro-2-methyl-1,3-dioxo-1H-pyrazolo[1,2-a]pyridazin-5-carboxylat als ölige Mischung von Diastereoisomeren. Die Diastereoisomeren werden durch Chromatographie getrennt,

wobei man Diastereoisomer A vom Schmelzpunkt 78-79°C (aus Essigester/Hexan) und Diastereoisomer B als farbloses Oel erhält.

(D)(b) In Analogie zu den Angaben in Beispiel 1 (D)(c) erhält man aus 5 g t-Butyl-hexahydro-2-methyl-1,3-dioxo-1H-pyrazolo[1,2-a]pyradazin-5-carboxylat und 6,19 g S-(2-Bromäthyl)thioacetat 4,22 g (61%) t-Butyl-2-(2-acetylthio-äthyl)-hexahydro-2-methyl-1,3-dioxo-1H-pyrazolo[1,2-a]-pyridazin-5-carboxylat als ölige Mischung von Diastereoisomeren. Durch Chromatographieren erhält man Diastereoisomer A vom Schmelzpunkt 79-80°C (aus Essigester/Hexan) und Diastereoisomer B vom Schmelzpunkt 67-70°C (aus Essigester/Hexan).

(D)(c) In Analogie zu den Angaben in Beispiel 1 (D)(c) erhält man aus 3,0 g t-Butyl-hexahydro-2-methyl-1,3-dioxo-1H-pyrazolo[1,2-a]pyridazin-5-carboxylat und 3,4 g S-(3-Brompropyl)thioacetat 2,18 g (51%) t-Butyl-2-(3-acetyl-thiopropyl)-hexahydro-2-methyl-1,3-dioxo-1H-pyrazolo-[1,2-a]pyridazin-5-carboxylat als ölige Mischung von Diastereoisomeren. Durch Chromatographieren erhält man Diastereoisomer A vom Schmelzpunkt 125-128°C (aus Essigester/Hexan) und Diastereoisomer B vom Schmelzpunkt 94-96°C (aus Essigester/Hexan).

## Beispiel 3

Man löst 1,5 g Diäthyl-5-t-butoxycarbonyl-hexahydro-1,3-dioxo-1H-pyrazolo[1,2-a]pyridazin-2,2-diacetat in 25 ml Trifluoressigsäure, lässt die erhaltene Lösung während 1 Stunde bei Raumtemperatur stehen, dampft die Mischung ein und kristallisiert den Rückstand aus Methylenchlorid/Hexan um. Man erhält 0,73 g (56%) Diäthyl-5-carboxy-hexahydro-1,3-dioxo-1H-pyrazolo[1,2-a]pyridazin-2,2-diacetat als beigen Festkörper vom Schmelzpunkt 105-108°C.

## Beispiel 4

(A) In Analogie zu den Angaben in Beispiel 1 (D)(a) erhält man aus t-Butyl-hexahydro-1,3-dioxo-1H-pyrazolo[1,2-a]pyridazin-5-carboxylat und Benzylbromacetat in 60-proz. Ausbeute Dibenzyl-5-t-butoxycarbonyl-hexahydro-1,3-dioxo-1H-pyrazolo[1,2-a]pyridazin-2,2-diacetat als farbloses Oel.

(B) In Analogie zu den Angaben in Beispiel 3 erhält man aus Dibenzyl-5-t-butoxycarbonyl-hexahydro-1,3-dioxo-1H-pyrazolo[1,2-a]pyridazin-2,2-diacetat Dibenzyl-5-carboxy-hexahydro-1,3-dioxo-1H-pyrazolo[1,2-a]pyridazin-2,2-diacetat als farbloses Oel.

(C) Durch Hydrieren einer Lösung der in Absatz (B) erhaltenen Säure in Methanol über Palladium/Kohle (10%) erhält man 5-Carboxy-hexahydro-1,3-dioxo-1H-pyrazolo[1,2-a]pyridazin-2,2-diessigsäure als farblosen hygroskopischen Festkörper (aus Essigester/Hexan).

## Beispiuel 5

In Analogie zu den Angaben in Beispiel 3 erhält man aus 0,3 g t-Butyl-2-(2-acetylthioäthyl)-hexahydro-2-methyl-1,3-dioxo-1H-pyrazolo[1,2-a]pyridazin-5-carboxylat (Diastereoisomer A) 0,25 g (98%) 2-(2-Acetylthioäthyl)-hexahydro-2-methyl-1,3-dioxo-1H-pyrazolo[1,2-a]pyridazin-5-carbonsäure (Diastereoisomer A) als weissen Festkörper vom Schmelzpunkt 142-143°C (aus Essigester).

## Beispiel 6

In Analogie zu den Angaben in Beispiel 3 erhält man aus 0,19 g t-Butyl-2-(3-acetylthiopropyl)-hexahydro-2-methyl-1,3-dioxo-1H-pyrazolo[1,2-a]pyridazin-5-carboxylat (Diastereoisomer A) 0,1 g (62%) 2-(3-Acetylthiopropyl)-hexahydro-2-methyl-1,3-dioxo-1H-pyrazolo[1,2-a]pyridazin-5-carbonsäure (Diastereoisomer A) als weissen Festkörper

vom Schmelzpunkt 160-161°C (aus Essigester/Hexan).

Beispiel 7

(A) Man erhitzt eine Mischung aus 120,8 g (0,4 Mol) Diäthyl-2-methyl-2-[2-(tetrahydro-2-pyranyloxy)äthyl]-propan-1,3-dioat und 150 ml wasserfreiem Hydrazin während 64 Stunden unter Rückfluss zum Sieden, destilliert das überschüssige Hydrazin ab und kristallisiert den Rückstand aus Methanol um. Man erhält 60,28 g (62%) 4-Methyl-4-[2-(tetrahydro-2-pyranyloxy)äthyl]pyrazolidin-3,5-dion vom Schmelzpunkt 179-180°C.

(B)(a) Man suspendiert 1,21 g (5 mMol) 4-Methyl-4-[2-(tetrahydro-2-pyranyloxy)äthyl]pyrazolidin-3,5-dion in 20 ml trockenem Dioxan, rührt die erhaltene Suspension bei Raumtemperatur unter einer Stickstoffatomsphäre, versetzt über einen Zeitraum von 15 Minuten mit einer Lösung von 0,543 g (5 mMol) t-Butylhypochlorit in 5 ml trockenem Dioxan, filtriert die erhaltene blaue Lösung und dampft das Filtrat im Vakuum ein. Man nimmt den Rückstand in 10 ml trockenem Dioxan auf und gibt diese Lösung unter Rühren portionenweise zu einer Lösung von 0,616 g (5,5 mMol) Methyl-penta-2,4-dienoat in 20 ml trockenem Dioxan, wobei man nach jeder Zugabe wartet, bis die blaue Farbe verschwindet. Nach beendeter Zugabe und Verschwinden der blauen Farbe rührt man die Lösung noch während 1 Stunde bei Raumtemperatur, dampft anschliessend im Vakuum ein und chromatographiert den Rückstand an Kieselgel. Durch Eluieren mit Diäthyläther erhält man 0,52 g (29%) Methyl-2,3,5,8-tetrahydro-2-methyl-1,3-dioxo-2-[2-(tetrahydro-2-pyranyloxy)-äthyl]-1H-pyrazolo[1,2-a]pyridazin-5-carboxylat vom Schmelzpunkt 127,5-128,5°C (aus Diäthyläther).

(B)(b) Man rührt eine Mischung von 14,52 g (0,06 Mol) 4-Methyl-4-[2-(tetrahydro-2-pyranyloxy)äthyl]pyrazolidin-3,5-dion und 6,72 g (0,066 Mol) Methyl-penta-2,4-dienoat in 150 ml trockenem Methylenchlorid unter einer Stickstoffatmosphäre bei Raumtemperatur und versetzt tropfenweise mit einer Lösung von 26,64 g (0,06 Mol) Bleitetraacetat in 100 ml trockenem Methylenchlorid, wobei die Zugabe so reguliert wird, dass die blaue Farbe jeweils wieder verschwindet. Nach beendeter Zugabe und nach Verschwinden der blauen Farbe filtriert man die Suspension, wäscht das Filtrat mit gesättigter wässriger Natriumbicarbonatlösung, trocknet über Magnesiumsulfat und dampft ein. Man erhält 8,12 g (38%) Methyl-2,3,5,8-tetrahydro-2-methyl-1,3-dioxo-2-[2-(tetrahydro-2-pyranyloxy)äthyl]-1H-pyrazolo[1,2-a]-pyridazin-5-carboxylat vom Schmelzpunkt 126,5-127,5°C (aus Diäthyläther).

(C) Man rührt eine Lösung von 3,52 g (0,01 Mol) Methyl-2,3,5,8-tetrahydro-2-methyl-1,3-dioxo-2-[2-(tetrahydro-2-pyranyloxy)äthyl]-1H-pyrazolo[1,2-a]pyridazin-5-carboxylat in 50 ml 1M-Salzsäure und 50 ml Methanol während 1,5 Stunden bei Raumtemperatur, entfernt das Lösungsmittel im Vakuum, nimmt den Rückstand in Methylenchlorid auf, trocknet über Magnesiumsulfat und dampft ein. Man erhält 2,6 g (97%) Methyl-2,3,5,8-tetrahydro-2-(2-hydroxyäthyl)-2-methyl-1,3-dioxo-1H-pyrazolo[1,2-a]pyridazin-5-carboxylat vom Schmelzpunkt 97-99°C (aus Diäthyläther).

(D) Man gibt 3,45 g (30 mMol) Methansulfonylchlorid unter Rühren tropfenweise zu einer auf 0°C gekühlten Lösung von 6,7 g (25 mMol) Methyl-2,3,5,8-tetrahydro-2-(2-hydroxyäthyl)-2-methyl-1,3-dioxo-1H-pyrazolo[1,2-a]pyridazin-5-carboxylat in 20 ml trockenem Pyridin, giesst die erhaltene Suspension nach 2 Stunden auf Eis/Wasser und stellt mit 2M-Salzsäure sauer. Durch Extraktion mit Methylenchlorid erhält man 7,84 g (75%) Methyl-2,3,5,8-tetrahydro-2-(2-methansulfonyloxyäthyl)-2-methyl-1,3-dioxo-1H-pyrazolo-[1,2-a]pyridazin-5-carboxylat vom Schmelzpunkt 104-105°C (aus Diäthyläther).

(E) Man erhitzt eine Mischung aus 7,6 g (22 mMol) Methyl-2,3,5,8-tetrahydro-2-(2-methansulfonyloxyäthyl)-2-methyl-1,3-dioxo-1H-pyrazolo[1,2-a]pyridazin-5-carboxylat und 6,6 g (44 mMol) Natriumjodid in 100 ml Aceton während 16 Stunden unter Rückfluss zum Sieden. Man filtriert die erhaltene Suspension, dampft das Filtrat zur Trockne ein und verteilt den Rückstand zwischen Methylenchlorid und Wasser. Die organische Phase wird mit 10-proz. wässrigem Natriumthiosulfat gewaschen, über Magnesiumsulfat getrocknet und eingedampft. Man erhält 6,7 g (80%) Methyl-2,3,5,8-tetrahydro-2-(2-jodäthyl)-2-methyl-1,3-dioxo-1H-pyrazolo[1,2-a]pyridazin-5-carboxylat vom Schmelzpunkt 86-87°C (aus Diäthyläther).

## Beispiel 8

Man rührt eine Mischung aus 6,43 g (17 mMol) Methyl-2,3,5,8-tetrahydro-2-(2-jodäthyl)-2-methyl-1,3-dioxo-1H-pyrazolo[1,2-a]pyridazin-5-carboxylat und 1,94 g (17 mMol) Kaliumthioacetat in 150 ml Aceton während 5 Stunden bei Raumtemperatur, dampft das Lösungsmittel ab und verteilt den Rückstand zwischen Methylenchlorid und Wasser. Die organische Phase wird abgetrennt, über Magnesiumsulfat getrocknet und eingedampft. Man erhält Methyl-2-(2-acetylthioäthyl)-2,3,5,8-tetrahydro-2-methyl-1,3-dioxo-1H-pyrazolo[1,2-a]pyridazin-5-carboxylat als Mischung von Diastereoisomeren. Durch Chromatographieren an Kieselgel unter Verwendung von Diäthyläther als Elutionsmittel erhält man 3,81 g (69%) Diastereoisomer A als farbloses Oel und 1,26 g (23%) Diastereoisomer B als farbloses Oel.

## Beispiel 9

Man lässt eine Lösung von 0,71 g t-Butyl-2-acetylthiomethyl-hexahydro-2-methyl-1,3-dioxo-1H-pyrazolo[1,2-a]-pyridazin-5-carboxylat (Diastereoisomer A) in 10 ml Trifluoressigsäure während 1 Stunde bei Raumtemperatur stehen, dampft die erhaltene Mischung zur Trockne ein und rührt

den Rückstand unter Stickstoff mit 50 ml einer 50:50-Mischung von Wasser und konzentriertem Ammoniak während 2 Stunden. Die Mischung wird mit Salzsäure auf pH 1 gestellt, mit Natriumchlorid gesättigt und mit Chloroform ausgeschüttelt. Die Chloroformauszüge werden über Natriumsulfat getrocknet und eingedampft. Den Rückstand kristallisiert man aus Essigester/Hexan um und erhält 0,22 g (43%) Hexahydro-2-mercaptomethyl-2-methyl-1,3-dioxo-1H-pyrazolo-[1,2-a]pyridazin-5-carbonsäure (Diastereoisomer A) als weisse Kristalle vom Schmelzpunkt 209-211°C.

Beispiel 10

In Analogie zu den Angaben in Beispiel 9 erhält man aus t-Butyl-2-(2-acetylthioäthyl)-hexahydro-2-methyl-1,3-dioxo-1H-pyrazolo[1,2-a]pyridazin-5-carboxylat (Diastereoisomer A) in 48-proz. Ausbeute Hexahydro-2-(2-mercaptoäthyl)-2-methyl-1,3-dioxo-1H-pyrazolo[1,2-a]pyridazin-5-carbonsäure (Diastereoisomer A) vom Schmelzpunkt 170-173°C (aus Essigester/Hexan).

Beispiel 11

In Analogie zu den Angaben in Beispiel 9 erhält man aus t-Butyl-2-(2-acetylthioäthyl)-hexahydro-2-methyl-1,3-dioxo-1H-pyrazolo[1,2-a]pyridazin-5-carboxylat (Diastereoisomer B) in 62-proz. Ausbeute Hexahydro-2-(2-mercaptoäthyl)-2-methyl-1,3-dioxo-1H-pyrazolo[1,2-a]pyridazin-5-carbonsäure (Diastereoisomer B) vom Schmelzpunkt 165-168°C (aus Essigester/Hexan).

Beispiel 12

In Analogie zu den Angaben in Beispiel 9 erhält man aus t-Butyl-2-(2-acetylthioäthyl)-hexahydro-1,3-dioxo-1H-pyrazolo[1,2-a]pyridazin-5-carboxylat in 53-proz. Ausbeute Hexahydro-2-(2-mercaptoäthyl)-1,3-dioxo-1H-pyrazolo[1,2-a]-pyridazin-5-carbonsäure als hygroskopischen Festkörper.

## Beispiel 13

In Analogie zu den Angaben in Beispiel 9 erhält man aus t-Butyl-2,2-bis(2-acetylthioäthyl)-hexahydro-1,3-dioxo-1H-pyrazolo[1,2-a]pyridazin-5-carboxylat in 90-proz. Ausbeute Hexahydro-2,2-bis(2-mercaptoäthyl)-1,3-dioxo-1H-pyrazolo[1,2-á]pyridazin-5-carbonsäure als farblosen Schaum.

## Beispiel 14

(A) In Analogie zu den Angaben in Beispiel 1(A) erhält man aus 6,5 g 1-Benzyloxycarbonylpiperazinsäure-t-butylester und 5 g Aethyl-äthylmalonylchlorid 5,5 g (54%) 1-Benzyl-3-t-butyl-2-(2-äthoxycarbonylbutyryl)hexahydropyridazin-1,3-dicarboxylat als Oel.

(B) In Analogie zu den Angaben in Beispiel 1(B) erhält man aus 5,5 g 1-Benzyl-3-t-butyl-2-(2-äthoxycarbonylbutyryl)hexahydropyridazin-1,3-dicarboxylat 2,7 g (69%) t-Butyl-2-(2-äthoxycarbonylbutyryl)hexahydrppyridazin-3-carboxylat als hellbraunen Festkörper vom Schmelzpunkt 78-79°C (aus Hexan).

(C) In Analogie zu den Angaben in Beispiel 1(C) erhält man aus 1,1 g t-Butyl-2-(2-äthoxycarbonylbutyryl)hexahydropyridazin-3-carboxylat 0,75 g (79%) t-Butyl-2-äthyl-hexahydro-1,3-dioxo-1H-pyrazolo[1,2-a]pyridazin-5-carboxylat als leicht bräunlichen Festkörper vom Schmelzpunkt 93-95°C (aus Chloroform/Hexan).

(D) In Analogie zu den Angaben in Beispiel 1(D)(c) erhält man aus 4 g t-Butyl-2-äthyl-hexahydro-1,3-dioxo-1H-pyrazolo-[1,2-a]pyridazin-5-carboxylat und 5,43 g S-(2-bromäthyl)-thioacetat t-Butyl-2-(2-acetylthioäthyl)-2-äthyl-hexahydro-1,3-dioxo-1H-pyrazolo[1,2-a]pyridazin-5-carboxylat als Mischung von Diastereoisomeren. Durch Chromatographieren erhält man 1,1 g (20%) Diastereoisomer A vom Schmelzpunkt 82-84°C (aus Diäthyläther/Hexan) und 0,4 g (7%) Diastereoisomer B als farbloses Oel.

(E) In Analogie zu den Angaben in Beispiel 3 erhält man aus 1,1 g t-Butyl-2-(2-acetylthioäthyl)-2-äthyl-hexahydro-1,3-dioxo-1H-pyrazolo[1,2-a]pyridazin-5-carboxylat (Diastereoisomer A) 0,7 g (75%) 2-(2-Acetylthioäthyl)-2-äthyl-hexahydro-1,3-dioxo-1H-pyrazolo[1,2-a]pyridazin-5-carbonsäure (Diastereoisomer A) als hellbraunen Festkörper vom Schmelzpunkt 110-111°C (aus Essigester/Hexan).

(F) Man erhitzt 0,33 g 2-(2-Acetylthioäthyl)-2-äthyl-hexahydro-1,3-dioxo-1H-pyrazolo[1,2-a]pyridazin-5-carbonsäure in 10 ml 2M-Salzsäure während 0,5 Stunden unter Rückfluss zum Sieden, dampft ein, nimmt den Rückstand in Methylenchlorid auf, trocknet die erhaltene Lösung über Magnesiumsulfat und dampft ein. Man erhält 0,27 g (94%) 2-Aethyl-hexahydro-2-(2-mercaptoäthyl)-1,3-dioxo-1H-pyrazolo[1,2-a]pyridazin-5-carbonsäure als farblosen Schaum.

## Beispiel 15

Man erhitzt 1,1 g Methyl-2-(2-acetylthioäthyl)-2,3,5,8-tetrahydro-2-methyl-1,3-dioxo-1H-pyrazolo[1,2-a]-pyridazin-5-carboxylat (Diastereoisomer A) in 25 ml 2M-Salzsäure während 3 Stunden unter Rückfluss zum Sieden, dampft ein, nimmt den Rückstand in Methylenchlorid auf, trocknet die erhaltene Lösung über Magnesiumsulfat und dampft ein. Man erhält 0,35 g (38%) 2,3,5,8-Tetrahydro-2-(2-mercaptoäthyl)-2-methyl-1,3-dioxo-1H-pyrazolo[1,2-a]-pyridazin-5-carbonsäure (Diastereoisomer A) als weissen Festkörper vom Schmelzpunkt 113-114°C (aus Diäthyläther/Petroläther).

## Beispiel 16

In Analogie zu den Angaben in Beispiel 15 erhält man aus 0,98 g Methyl-2-(2-acetylthioäthyl)-2,3,5,8-tetrahydro-2-methyl-1,3-dioxo-1H-pyrazolo[1,2-a]pyridazin-5-carboxylat

(Diastereoisomer B) 0,56 g (69%) 2,3,5,8-Tetrahydro-2-(2-mercaptoäthyl)-2-methyl-1,3-dioxo-1H-pyrazolo[1,2-a]-pyridazin-5-carbonsäure (Diastereoisomer B) als schwach gelben Schaum.

Beispiel 17

(A) In Analogie zu den Angaben in Beispiel 7(A) erhält man aus 66,5 g Diäthyl-2-benzyl-2-[2-(tetrahydro-2-pyranyloxy)äthyl]-propan-1,3-dioat und 110 ml wasserfreiem Hydrazin 34,6 g (62%) 4-Benzyl-2-[2-(tetrahydro-2-pyranyloxy)äthyl]pyrazolidin-3,5-dion als weissen Festkörper vom Schmelzpunkt 198-200°C (aus Methanol).

(B) In Analogie zu den Angaben in Beispiel 7(B)(b) erhält man aus 2,7 g 4-Benzyl-4-[2-(tetrahydro-2-pyranyloxy)-äthyl]pyrazolidin-3,5-dion und 1,05 g Methyl-penta-2,4-dienoat 1,2 g (34%) Methyl-2-benzyl-2,3,5,8-tetrahydro-1,3-dioxo-2-[2-(tetrahydro-2-pyranyloxy)äthyl]-1H-pyrazolo-[1,2-a]pyridazin-5-carboxylat als weissen Festkörper vom Schmelzpunkt 113-114°C (aus Diäthyläther).

(C) In Analogie zu den Angaben in Beispiel 7(C) erhält man aus 1 g Methyl-2-benzyl-2,3,5,8-tetrahydro-1,3-dioxo-2-[2-(tetrahydro-2-pyranyloxy)äthyl]-1H-pyrazolo[1,2-a]-oyridazin-5-carboxylat 0,71 g (88%) Methyl-2-benzyl-2,3,5,8-tetrahydro-2-(2-hydroxyäthyl)-2,3-dioxo-1H-pyrazolo[1,2-a]-pyridazin-5-carboxylat als weissen Festkörper vom Schmelzpunkt 148-149°C (aus Diäthyläther).

(D) In Analogie zu den Angaben in Beispiel 7(D) erhält man aus 6,4 g Methyl-2-benzyl-2,3,5,8-tetrahydro-2-(2-hydroxyäthyl)-1,3-dioxo-1H-pyrazolo[1,2-a]pyridazin-5-carboxylat und 2,6 g Methansulfonyl-chlorid 5,5 g (71%) Methyl-2-benzyl-2,3,5,8-tetrahydro-2-(2-methansulfonyloyäthyl)-1,3-dioxo-1H-pyrazolo[1,2-a]pyridazin-5-carboxylat als weissen Festkörper vom Schmelzpunkt 161-163°C (aus Diäthyläther).

(E) In Analogie zu den Angaben in Beispiel 7(E) erhält man aus 5,9 g Methyl-2-benzyl-2,3,5,8-tetrahydro-2-(2-methansulfonyloxyäthyl)-1,3-dioxo-1H-pyrazolo[1,2-a]pyridazin-5-carbonsäure und 4,2 g Natriumjodid 3,25 g (51%) Methyl-2-benzyl-2,3,5,8-tetrahydro-2-(2-jodäthyl)-1,3-dioxo-1H-pyrazolo[1,2-a]pyridazin-5-carboxylat als weissen Festkörper vom Schmelzpunkt 90-92°C (aus Diäthyläther).

(F)(a) Man rührt eine Mischung aus 5,2 g Methyl-2-benzyl-2,3,5,8-tetrahydro-2-(2-jodäthyl)-1,3-dioxo-1H-pyrazolo-[1,2-a]pyridazin-5-carboxylat und 1,31 g Kaliumthioacetat in 150 ml Aceton während 8 Stunden bei Raumtemperatur, dampft das Lösungsmittel ab und verteilt den Rückstand zwischen Methylenchlorid und Wasser. Die organische Phase wird abgetrennt, über Magnesiumsulfat getrocknet und eingedampft. Man erhält Methyl-2-(2-acetylthioäthyl)-2-benzyl-2,3,5,8-tetrahydro-1,3-dioxo-1H-pyrazolo[1,2-a]pyridazin-5-carboxylat als Mischung von Diastereoisomeren. Durch Verreiben mit Diäthyläther erhält man 3,5 g Diastereoisomer B als weissen Festkörper vom Schmelzpunkt 161-162°C (aus Essigester).

(F)(b) Man rührt eine Mischung aus 0,5 g Methyl-2-benzyl-2,3,5,8-tetrahydro-2-(2-jodäthyl)-1,3-dioxo-1H-pyrazolo-[1,2-a]pyridazin-5-carboxylat, 0,15 g Kaliumacetat und 0,1 g Thioessigsäure in 40 ml Aceton während 5 Stunden bei Raumtemperatur, dampft das Lösungsmittel ab und verteilt den Rückstand zwischen Methylenchlorid und Wasser. Die organische Phase wird abgetrennt, über Magnesiumsulfat getrocknet und eingedampft. Man erhält 0,4 g (90%) Methyl-2-(2-acetylthioäthyl)-2-benzyl-2,3,5,8-tetrahydro-1,3-dioxo-1H-pyrazolo[1,2-a]pyridazin-5-carboxylat (Diastereoisomer A) als schwach gelbes Oel.

(G)(a) In Analogie zu den Angaben in Beispiel 15 erhält man aus 1,6 g Methyl-2-(2-acetylthioäthyl)-2-benzyl-2,3,5,8-tetrahydro-1,3-dioxo-1H-pyrazolo[1,2-a]pyridazin-5-carboxylat (Diastereoisomer B) 0,68 g (49%) 2-Benzyl-2,3,5,8-tetrahydro-2-(2-mercaptoäthyl)-1,3-dioxo-1H-pyrazolo[1,2-a]pyridazin-5-carbonsäure (Diastereoisomer B) als weissen Festkörper vom Schmelzpunkt 151-154°C (aus Essigester).

(G)(b) In Analogie zu den Angaben in Beispiel 15 erhält man aus 0,8 g Methyl-2-(2-acetylthioäthyl)-2-benzyl-2,3,5,8-tetrahydro-1,3-dioxo-1H-pyrazolo[1,2-a]pyridazin-5-carboxylat (Diastereoisomer A) 0,25 g (36%) 2-Benzyl-2,3,5,8-tetrahydro-2-(2-mercaptoäthyl)-1,3-dioxo-1H-pyrazolo[1,2-a]-pyridazin-5-carbonsäure (Diastereoisomer A) als weissen Festkörper vom Schmelzpunkt 184-185°C (aus Diäthyläther).

## Beispiel 18

(A) In Analogie zu den Angaben in Beispiel 7(A) erhält man aus 6,32 g Diäthyl-2-äthyl-2-[2-(tetrahydro-2-pyranyloxy)-äthyl]-propan-1,3-dioat und 10,5 ml wasserfreiem Hydrazin 2,3 g (45%) 4-Aethyl-4-[2-(tetrahydro-2-pyranyloxy)äthyl]-pyrazolidin-3,5-dion als weissen Festkörper vom Schmelzpunkt 188-191°C (aus Methanol).

(B) In Analogie zu den Angaben in Beispiel 7(B)(b) erhält man aus 7,75 g 4-Aethyl-4-[2-(tetrahydro-2-pyranyloxy)-äthyl]pyrazolidin-3,5-dion und 3,7 g Methyl-penta-2,4-dienoat 6,49 g (59%) Methyl-2-äthyl-2,3,5,8-tetrahydro-1,3-dioxo-2-[2-(tetrahydro-2-pyranyloxy)äthyl]-1H-pyrazolo-[1,2-a]pyridazin-5-carboxylat als weissen Festkörper vom Schmelzpunkt 96-97°C (aus Diäthyläther).

(C) In Analogie zu den Angaben in Beispiel 7(C) erhält man aus 16,5 g Methyl-2-äthyl-2,3,5,8-tetrahydro-1,3-dioxo-2-[2-(tetrahydro-2-pyranyloxy)äthyl]-1H-pyrazolo[1,2-a]-pyridazin-5-carboxylat 6,88 g (54%) Methyl-2-äthyl-2,3,5,8-tetrahydro-2-(2-hydroxyäthyl)-1,3-dioxo-1H-pyrazolo[1,2-a]-pyridazin-5-carboxylat als weissen Festkörper vom Schmelzpunkt 87-92°C (aus Diäthyläther).

(D) In Analogie zu den Angaben in Beispiel 7(D) erhält man aus 6,30 g Methyl-2-äthyl-2,3,5,8-tetrahydro-2-(2-hydroxyäthyl)-1,3-dioxo-1H-pyrazolo[1,2-a]pyridazin-5-carboxylat 5,14 g (65%) Methyl-2-äthyl-2,3,5,8-tetrahydro-2-(2-methan-sulfonyloxyäthyl)-1,3-dioxo-1H-pyrazolo[1,2-a]pyridazin-5-carboxylat als weissen Festkörper vom Schmelzpunkt 94-96°C (aus Diäthyläther).

(E) In Analogie zu den Angaben in Beispiel 7(E) erhält man aus 8,22 g Methyl-2-äthyl-2,3,5,8-tetrahydro-2-(2-methan-sulfonyloxyäthyl)-1,3-dioxo-1H-pyrazolo[1,2-a]pyridazin-5-carboxylat 5 g (55%) Methyl-2-äthyl-2,3,5,8-tetrahydro-2-(2-jodäthyl)-1,3-dioxo-1H-pyrazolo[1,2-a]pyridazin-5-carboxylat als weissen Festkörper vom Schmelzpunkt 95-97°C (aus Diäthyläther/Hexan).

(F) In Analogie zu den Angaben in Beispiel 17(F)(b) erhält man aus 3,25 g Methyl-2-äthyl-2,3,5,8-tetrahydro-2-(2-jodäthyl)-1,3-dioxo-1H-pyrazolo[1,2-a]pyridazin-5-carboxylat 2,5 g (89%) Methyl-2-(2-acetylthioäthyl)-2-äthyl-2,3,5,8-tetrahydro-1,3-dioxo-1H-pyrazolo[1,2-a]pyridazin-5-carboxylat (Diastereoisomer A) als schwach gelbes Oel.

(G) In Analogie zu den Angaben in Beispiel 15 erhält man aus 0,5 g Methyl-2-(2-acetylthioäthyl)-2-äthyl-2,3,5,8-tetrahydro-1,3-dioxo-1H-pyrazolo[1,2-a]pyridazin-5-carboxylat (Diastereoisomer A) 0,21 g (49%) 2-Aethyl-2,3,5,8-tetrahydro-2-(2-mercaptoäthyl)-1,3-dioxo-1H-pyrazolo[1,2-a]-pyridazin-5-carbonsäure (Diastereoisomer A) als weissen Schaum.

## Beispiel 19

(A) Man rührt 1,4 g Kaliumcarbonat, 2,68 g t-Butyl-hexahydro-2-methyl-1,3-dioxo-1H-pyrazolo[1,2-a]pyridazin-5-carboxylat und 3,6 g Phenyl-bromacetat in 20 ml Aceton während 7 Stunden unter Rühren unter Rückfluss zum Sieden. Man filtriert, dampft ein und chromatographiert das erhaltene Oel an 300 g Kieselgel unter Eluieren mit 5% Essigester in Methylenchlorid. Man erhält nach Kristallisieren aus Essigester/Hexan 1,83 g Phenyl-5-t-butoxycarbonyl-hexahydro-2-methyl-1,3-dioxo-1H-pyrazolo[1,2-a]pyridazin-2-acetat vom Schmelzpunkt 139-141°C.

(B) Man behandelt 2,2 g Phenyl-5-t-butoxycarbonyl-hexahydro-2-methyl-1,3-dioxo-1H-pyrazolo[1,2-a]pyridazin-2-acetat in 12 ml 1,2-Dimethoxyäthan mit 9 ml methanolischem Hydroxylamin (hergestellt, indem man 1,38 g Hydroxylaminhydrochlorid in Methanol auflöst, mit 1,12 g Kaliumhydroxid-Plättchen versetzt, mit Methanol auf 14 ml ergänzt und die Lösung vor der Verwendung filtriert). Nach 18 Stunden entfernt man das Lösungsmittel, versetzt mit 300 ml Essigester und wäscht die Mischung zuerst mit verdünnter Natriumchloridlösung und anschliessend mit gesättiger Natriumchloridlösung. Die organische Phase wird über Magnesiumsulfat getrocknet und eingedampft. Nach Kristalliseren des erhaltenen Oels aus Essigester erhält man 0,98 g t-Butyl-hexahydro-2-[(N-hydroxycarbamoyl)methyl]-2-methyl-1,3-dioxo-1H-pyrazolo[1,2-a]pyridazin-5-carboxylat vom Schmelzpunkt 157-165°C (Zersetzung).

(C) Man behandelt 0,8 g t-Butyl-hexahydro-2-[(N-hydroxycarbamoyl)methyl]-2-methyl-1,3-dioxo-1H-pyrazolo[1,2-a]-pyridazin-5-carboxylat während 1 Stunde mit 10 ml Trifluoressigsäure, dampft anschliessend ein, behandelt den Rückstand mit Essigester und dampft erneut ein. Durch Verreiben des Rückstandes mit 1,2-Dimethoxyäthan und Umkristallisieren aus Methanol/Wasser erhält man 0,21 g Hexahydro-2-[(N-hydroxycarbamoyl)methyl]-2-methyl-1,3-dioxo-1H-pyrazolo-[1,2-a]pyridazin-5-carbonsäure vom Schmelzpunkt 254°C (Zersetzung).

Beispiel 20

(A) Man behandelt eine Lösung von 27 g 1-Benzyloxycarbonylpiperazinsäure-t-butylester in 500 ml Methylenchlorid mit 250 ml gesättigter wässriger Natriumbicarbonatlösung, 30 g Natriumbromid in 50 ml Wasser und mit 28,6 g 3-Brom-2-brommethylpropanoylchlorid. Man rührt die Mischung während 18 Stunden bei Raumtemperatur, trennt die organische Phase ab, trocknet über Magnesiumsulfat und dampft ein. Der Rückstand wird an 400 g Kieselgel chromatographiert, wobie man 2-5% Essigester in Toluol als Elutionsmittel verwendet. Man erhält nach Umkristallisieren aus Essigester/Hexan 27,3 g (59%) 1-Benzyl-3-t-butyl-2-(3-brom-2-brommethylpropanoyl)-hexahydropyridazin-1,3-dicarboxylat vom Schmelzpunkt 101-102°C.

(B) Man hydriert 13,3 g 1-Benzyl-3-t-butyl-2-(3-brom-2-brommethylpropanoyl)-hexahydropyridazin-1,3-dicarboxylat in 122 ml Essigsäure und 122 ml Methanol in Gegenwart von 1,4 g Palladium/Kohle, wobei die Apparatur mit einem Natronkalk-Röhrchen versehen wird. Man filtriert den Katalysator ab, behandelt das Filtrat mit 3,2 g Natriumacetattrihydrat und dampft ein. Man extrahiert den Rückstand mit 300 ml Essigester und chromatographiert diese Lösung an 400 g Kieselgel unter Verwendung von Diäthyläther/Hexan als Elutionsmittel. Man erhält 1,25 g t-Butyl-2-brommethylhexahydro-3-oxo-1H-pyrazolo[1,2-a]pyridazin-5-carboxylat (Diastereoisomer A) vom Schmelzpunkt 109-111°C (aus Hexan), 2,45 g t-Butyl-2-brommethyl-hexahydro-3-oxo-1H-pyrazolo-[1,2-a]pyridazin-5-carboxylat (Diastereoisomer B) vom Schmelzpunkt 88-89°C (aus Hexan) und 1,20 g einer Mischung der oben erwähnten Diastereoisomeren.

(C) Man rührt 1,42 g t-Butyl-2-brommethyl-hexahydro-3-oxo-1H-pyrazolo[1,2-a]pyridazin-5-carboxylat (Diasteroisomer A) und 0,58 g Kaliumthioacetat in 20 ml Aceton während 17 Stunden bei Raumtemperatur, dampft das Lösungsmittel ab, verdünnt den Rückstand mit 100 ml Essigester, wäscht die erhaltene Mischung dreimal mit Natriumchloridlösung, trocknet über Magnesiumsulfat und dampft ein. Nach Umkristallisieren des Rückstandes aus Essigester/Hexan erhält man 1,07 g t-Butyl-2-acetylthiomethyl-hexahydro-3-oxo-1H-pyrazolo-[1,2-a]pyridazin-5-carboxylat (Diastereoisomer A) vom Schmelzpunkt 92-93°C (aus Essigester/Hexan).

Beispiel 21

Man gibt 2,3 ml 1,5-Diazabicyclo[5.4.0]undec-5-en in 10 ml Dioxan bei 5°C zu einer Lösung von 4,4 g t-Butyl-2-brommethyl-hexahydro-3-oxo-1H-pyrazolo[1,2-a]oyridazin-5-carboxylat (Diastereoisomer B) in 25 ml Dioxan, lässt die Mischung während 2 Stunden bei Raumtemperatur stehen, filtriert, dampft das Filtrat ein und nimmt den Rückstand in Essigester auf. Die erhaltene Lösung wird über 6 g Kieselgel filtriert und eingedampft. Man nimmt den erhaltenen Rückstand in 10 ml Aceton auf, behandelt die Lösung mit 1,6 g Kaliumthioacetat und anschliessend mit 10 ml Thioessigsäure und rührt die erhaltene Mischung über Nacht bei Raumtemperatur. Man verdünnt mit 100 ml Methylenchlorid, wäscht mit 5 ml gesättigter wässriger Natriumbicarbonatlösung, trocknet die abgetrennte organische Phase über Magnesiumsulfat und dampft ein. Das erhaltene Oel wird an 200 g Kieselgel chromatographiert unter Verwendung von Diäthyläther/Hexan als Elutionsmittel. Nach Umkristallisieren aus Essigester/Hexan erhält man 1,69 g t-Butyl-2-acetylthiomethyl-hexahydro-3-oxo-1H-pyrazolo[1,2-a]pyridazin-5-carboxylat (Diastereoisomer A) und 0,9 g des entsprechenden Diastereoisomeren B.

Beispiel 22

Man behandelt 1,69 g t-Butyl-2-acetylthiomethyl-hexahydro-3-oxo-1H-pyrazolo[1,2-a]pyridazin-5-carboxylat (Diastereoisomer A) bei Raumtemperatur und während 2 Stunden mit 10 ml Trifluoressigsäure. Man dampft die Lösung ein und versetzt den Rückstand mehrmals mit Toluol, wobei man jeweils

wieder eindampft. Durch Kristallisieren des Rückstandes aus Aceton erhält man 1,1 g 2-Acetylthiomethyl-hexahydro-3-oxo-1H-pyrazolo[1,2-a]pyridazin-5-carbonsäure vom Schmelzpunkt 166-168°C (Zersetzung).

Beispiel 23

Man erhitzt 1,8 g 2-Acetylthiomethyl-hexahydro-3-oxo-1H-pyrazolo[1,2-a]pyridazin-5-carbonsäure in 2M-Salzsäure während 1,5 Stunden auf 95°C, kühlt die Mischung ab, stellt den pH der Lösung mit Dinatrium-hydrogen-orthophosphat auf 3 ein und extrahiert die wässrige Phase mehrmals mit Essigester. Die organischen Auszüge werden über Magnesiumsulfat getrocknet und eingedampft. Nach Umkristallisieren des Rückstandes aus Aceton erhält man 1,1 g Hexahydro-2-mercaptomethyl-3-oxo-1H-pyrazolo[1,2-a]pyridazin-5-carbonsäure vom Schmelzpunkt 145,5-147°C.

Beispiel 24

(A) Man hydriert eine Mischung aus 6,4 g 1-Benzyloxycarbonylpiperazinsäure-t-butylester und 2,56 g 3-Methyldihydro-2(3H)-furanon-3-ylcarboxaldehyd (J. Med.Chem. 1976, 19 309-313) in 200 ml Methanol über 0,9 g Palladium/Kohle (10%) bei Raumtemperatur und Atmosphärendruck, filtriert anschliessend den Katalysator ab, dampft das Filtrat ein und chromatographiert den Rückstand an Kieselgel. Man erhält 3,3 g (56%) 6-Dimethyläthyl-4a-methyl-4-oxo-2,3,4a,4,6,7,8,9-octahydro-furo[2,3-c]pyrazolo[1,2-a]pyridazin-6-carboxylat (Diastereoisomer A) als gelbliches Oel.

(B) Man löst 1,62 g 6-Dimethyläthyl-4a-methyl-4-oxo-2,3,4a,-4,6,7,8,9-octahydro-furo[2,3-c]pyrazolo[1,2-a]pyridazin-6-carboxylat (Diastereoisomer A) in 25 ml Wasser und 10 ml Dioxan, behandelt die Lösung mit 2,75 ml 2M-Salzsäure und hydriert die erhaltene Mischung über 160 ml Palladium/Kohle (10%) bei Raumtemperatur und Atosphärendruck. Man filtriert den Katalysator ab, dampft das Filtrat ein, nimmt den

Rückstand in Pyridin auf und dampft erneut ein. Man nimmt den Rückstand in 15 ml Pyridin auf, behandelt bei 0°C tropfenweise mit 1,27 ml Methansulfonylchlorid, rührt die erhaltene Mischung während 2 Stunden bei 0°C, dampft die entstandene Suspension ein und verteilt den Rückstand zwischen 50 ml Chloroform und 10 ml 2M-Salzsäure. Man wäscht die organische Phase mit Natriumchloridlösung und chromatographiert den erhaltenen Rückstand an Kieselgel. Man erhält 0,77 g (37%) t-Butyl-hexahydro-2-(2-methansulfonyloxyäthyl)-2-methyl-3-oxo-1H-pyrazolo[1,2-a]pyridazin-5-carboxylat (Diastereoisomer A) als Oel.

(C) Man behandelt eine Lösung von 0,77 g t-Butyl-hexahydro-2-(2-methansulfonyloxyäthyl)-2-methyl-3-oxo-1H-pyrazolo[1,2-a]pyridazin-5-carboxylat (Diastereoisomer A) in 20 ml Aceton mit 0,3 g Natriumjodid und 0,23 g Kaliumthioacetat, rührt die Mischung während 24 Stunden bei Raumtemperatur und dampft anschliessend ein. Nach Chromatographieren des Rückstandes an Kieselgel erhält man 0,43 g (59%) t-Butyl-hexahydro-2-(2-acetylthioäthyl)-2-methyl-3-oxo-1H-pyrazolo[1,2-a]pyridazin-5-carboxylat (Diastereoisomer A) als Oel.

(D) Man lässt 0,43 g t-Butyl-hexahydro-2-(2-acetylthioäthyl)-2-methyl-3-oxo-1H-pyrazolo[1,2-a]pyridazin-5-carboxylat (Diastereoisomer A) in 2 ml Trifluoressigsäure während 2 Stunden bei Raumtemperatur stehen, dampft die Mischung zur Trockne ein und rührt den Rückstand unter Stickstoff in 6 ml Wasser/konzentriertem Ammoniak (50:50) während 2 Stunden. Man stellt den pH der Mischung mit Salzsäure auf 3 ein, sättigt mit Natriumchlorid und extrahiert mit Chloroform. Durch Chromatographieren an Kieselgel erhält man 0,16 g eines weisslichen Festkörpers. Durch Umkristallisieren aus Essigester/Hexan erhält man 0,1 g (32%) Hexahydro-2-(2-mercaptoäthyl)-2-methyl-3-oxo-1H-pyrazolo[1,2-a]pyridazin-5-carbonsäure (Diastereoisomer A) als weissliche Kristalle vom Schmelzpunkt 119-122°C.

Die folgenden Beispiele beschreiben pharmazeutische Präparate, enthaltend ein erfindungsgemässes Pyrazolopyridazin-Derivat:

## Beispiel A

In herkömmlicher Weise werden Tabletten folgender Zusammensetzung hergestellt:

| Bestandteile | | per/Tablette |
|---|---|---|
| Pyrazolopyridazin-Derivat | | 10,0 mg |
| Lactose | | 125,0 mg |
| Maisstärke | | 75,0 mg |
| Talk | | 4,0 mg |
| Magnesiumstearat | | 1,0 mg |
| | Gesamtgewicht | 215,0 mg |

## Beispiel B

In herkömmlicher Weise werden Kapseln folgender Zusammensetzung hergestellt:

| Bestandteile | | per/Tablette |
|---|---|---|
| Pyrazolopyridazin-Derivat | | 25,0 mg |
| Lactose | | 150,0 mg |
| Maisstärke | | 20,0 mg |
| Talk | | 5,0 mg |
| | Gesamtgewicht | 200,0 mg |

Patentansprüche

1. Pyrazolopyridazin-Derivate der allgemeinen Formel

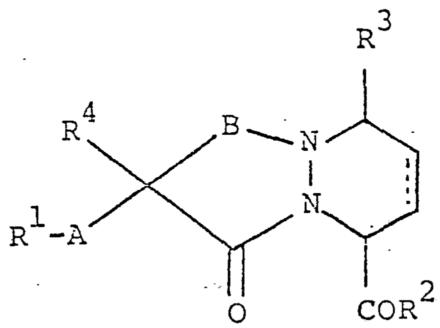

I

worin A gegebenenfalls durch niederes Alkyl substituiertes Methylen, Aethylen oder Propylen, B eine Carbonyl- oder Methylengruppe, $R^1$ Halogen, Carboxyl, niederes Alkoxycarbonyl, Hydroxyaminocarbonyl, Mercapto, niederes Alkanoylthio oder Aryl-niederes Alkylthio, $R^2$ Hydroxy, niederes Alkoxy oder Amino und $R^3$ Wasserstoff, niederes Alkyl, Aryl oder Aryl-niederes Alkyl bedeuten, $R^4$ Wasserstoff, niederes Alkyl, Aryl, Aryl-niederes Alkyl oder die Gruppe $-A-R^1$ bedeutet, wobei A und $R^1$ obige Bedeutung besitzen, und die gestrichelte Linie eine fakultative Bindung bedeutet, wenn B eine Carbonylgruppe bedeutet,

und Salze von Verbindungen der allgemeinen Formel I, worin $R^1$ Carboxy und/oder $R^2$ Hydroxy bedeuten, mit pharmazeutisch annehmbaren Basen.

2. Verbindungen gemäss Anspruch 1, dadurch gekennzeichnet, dass B eine Carbonylgruppe bedeutet.

3. Verbindungen gemäss Anspruch 1 oder 2, dadurch gekennzeichnet, dass $R^1$ Mercapto bedeutet.

4. Verbindungen gemäss einem der Ansprüche 1 bis 3, dadurch gekennzeichnet, dass $R^2$ Hydroxy bedeutet.

5. Verbindungen gemäss einem der Ansprüche 1 bis 4, dadurch gekennzeichnet, dass $R^3$ Wasserstoff bedeutet.

6. Verbindungen gemäss einem der Ansprüche 1 bis 5, dadurch gekennzeichnet, dass $R^4$ Wasserstoff, niederes Alkyl, vorzugsweise Methyl oder Aethyl, oder Aryl-niederes Alkyl, vorzugsweise Benzyl, bedeutet.

7. Verbindungen gemäss einem der Ansprüche 1 bis 6, dadurch gekennzeichnet, dass B eine Carbonylgruppe, $R^1$ Mercapto, $R^2$ Hydroxy, $R^3$ Wasserstoff und $R^4$ Wasserstoff, Methyl, Aethyl oder Benzyl bedeuten.

8. 2,3,5,8-Tetrahydro-2-(2-mercaptoäthyl)-2-methyl-1,3-dioxo-1H-pyrazolo[1,2-a]pyridazin-5-carbonsäure.

9. Hexahydro-2-mercaptomethyl-2-methyl-1,3-dioxo-1H-pyrazolo[1,2-a]pyridazin-5-carbonsäure, Hexahydro-2-(2-mercaptoäthyl)-2-methyl-1,3-dioxo-1H-pyrazolo[1,2-a]-pyridazin-5-carbonsäure, Hexahydro-2-(2-mercaptoäthyl)-1,3-dioxo-1H-pyrazolo[1,2-a]pyridazin-5-carbonsäure und Hexahydro-2,2-bis(2-mercaptoäthyl)-1,3-dioxo-1H-pyrazolo-[1,2-a]pyridazin-5-carbonsäure.

10. 2-Aethyl-hexahydro-2-(2-mercaptoäthyl)-1,3-dioxo-1H-pyrazolo[1,2-a]pyridazin-5-carbonsäure, 2-Benzyl-2,3,5,8-tetrahydro-2-(2-mercaptoäthyl)-1,3-dioxo-1H-pyrazolo[1,2-a]-pyridazin-5-carbonsäure, 2-Aethyl-2,3,5,8-tetrahydro-2-(2-mercaptoäthyl)-1,3-dioxo-1H-pyrazolo[1,2-a]pyridazin-5-carbonsäure, Hexahydro-2-mercaptomethyl-3-oxo-1H-pyrazolo-[1,2-a]pyridazin-5-carbonsäure und Hexahydro-2-(2-mercapto-äthyl)-2-methyl-3-oxo-1H-pyrazolo[1,2-a]pyridazin-5-carbonsäure.

11. Diäthyl-5-t-butoxycarbonyl-hexahydro-1,3-dioxo-1H-pyrazolo[1,2-a]pyridazin-2,2-diacetat, 2,2-Bis(2-äthoxy-carbonyläthyl)-hexahydro-1,3-dioxo-1H-pyrazolo[1,2-a]pyridazin-5-carboxylat, t-Butyl-2,2-bis(acetylthiomethyl)-hexahydro-1,3-dioxo-1H-pyrazolo[1,2-a]pyridazin-5-carboxylat, t-Butyl-2,2-bis(2-acetylthioäthyl)-hexahydro-1,3-dioxo-1H-pyrazolo[1,2-a]pyridazin-5-carboxylat, t-Butyl-2-(2-acetyl-

thioäthyl)-hexahydro-1,3-dioxo-1H-pyrazolo[1,2-a]pyridazin-5-carboxylat, t-Butyl-2-acetylthiomethyl-hexahydro-2-methyl-1,3-dioxo-1H-pyrazolo[1,2-a]pyridazin-5-carboxylat, t-Butyl-2-(2-acetylthioäthyl)-hexahydro-2-methyl-1,3-dioxo-1H-pyrazolo[1,2-a]pyridazin-5-carboxylat, t-Butyl-2-(3-acetylthiopropyl)-hexahydro-2-methyl-1,3-dioxo-1H-pyrazolo-[1,2-a]pyridazin-5-carboxylat, Diäthyl-5-carboxy-hexahydro-1,3-dioxo-1H-pyrazolo[1,2-a]pyridazin-2,2-diacetat, 5-Carboxy-hexahydro-1,3-dioxo-1H-pyrazolo[1,2-a]pyridazin-2,2-diessigsäure, 2-(2-Acetylthioäthyl)-hexahydro-2-methyl-1,3-dioxo-1H-pyrazolo[1,2-a]pyridazin-5-carbonsäure, 2-(3-Acetylthiopropyl)-hexahydro-2-methyl-1,3-dioxo-1H-pyrazolo-[1,2-a]pyridazin-5-carbonsäure, Methyl-2,3,5,8-tetrahydro-2-(2-jodäthyl)-2-methyl-1,3-dioxo-1H-pyrazolo[1,2-a]-pyridazin-5-carboxylat und Methyl-2-(2-acetylthioäthyl)-2,3,5,8-tetrahydro-2-methyl-1,3-dioxo-1H-pyrazolo[1,2-a]-pyridazin-5-carboxylat.

12. t-Butyl-2-(2-acetylthioäthyl)-2-äthyl-hexahydro-1,3-dioxo-1H-pyrazolo[1,2-a]pyridazin-5-carboxylat, 2-(2-Acetylthioäthyl)-2-äthyl-hexahydro-1,3-dioxo-1H-pyrazolo-[1,2-a]pyridazin-5-carbonsäure, Methyl-2-benzyl-2,3,5,8-tetrahydro-2-(2-jodäthyl)-1,3-dioxo-1H-pyrazolo[1,2-a]-pyridazin-5-carboxylat, Methyl-2-(2-acetylthioäthyl)-2-benzyl-2,3,5,8-tetrahydro-1,3-dioxo-1H-pyrazolo[1,2-a]-pyridazin-5-carboxylat, Methyl-2-äthyl-2,3,5,8-tetrahydro-2-(2-jodäthyl)-1,3-dioxo-1H-pyrazolo[1,2-a]pyridazin-5-carboxylat, Methyl-2-(2-acetylthioäthyl)-2-äthyl-2,3,5,8-tetrahydro-1,3-dioxo-1H-pyrazolo[1,2-a]pyridazin-5-carboxylat, t-Butyl-hexahydro-2-[(N-hydroxycarbamoyl)methyl]-2-methyl-1,3-dioxo-1H-pyrazolo[1,2-a]pyridazin-5-carboxylat, Hexahydro-2-[(N-hydroxycarbamoyl)methyl]-2-methyl-1,3-dioxo-1H-pyrazolo[1,2-a]pyridazin-5-carbonsäure, t-Butyl-2-brommethyl-hexahydro-3-oxo-1H-pyrazolo[1,2-a]pyridazin-5-carboxylat, t-Butyl-2-acetylthiomethyl-hexahydro-3-oxo-1H-pyrazolo[1,2-a]pyridazin-5-carboxylat, 2-Acetylthiomethyl-hexahydro-3-oxo-1H-pyrazolo[1,2-a]pyridazin-5-carbonsäure und t-Butyl-hexahydro-2-(2-acetylthioäthyl)-2-methyl-3-oxo-1H-pyrazolo[1,2-a]pyridazin-5-carboxylat.

13. Verbindungen der allgemeinen Formel

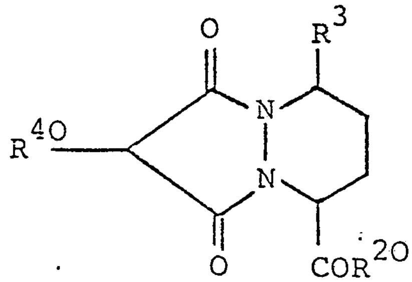

II

worin $R^3$ Wasserstoff, niederes Alkyl, Aryl oder Aryl-niederes Alkyl, $R^{20}$ niederes Alkoxy und $R^{40}$ Wasserstoff, niederes Alkyl, Aryl oder Aryl-niederes Alkyl bedeuten.

14. Verbindungen der allgemeinen Formel

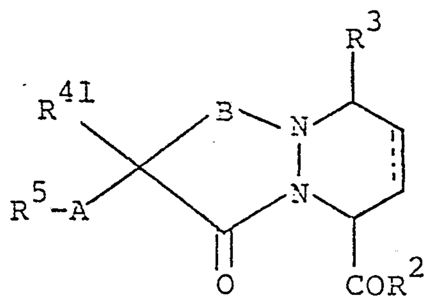

IV

worin A gegebenenfalls durch niederes Alkyl substituiertes Methylen, Aethylen oder Propylen, B eine Carbonyl- oder Methylengruppe, $R^2$ Hydroxy, niederes Alkoxy oder Amino, $R^3$ Wasserstoff, niederes Alkyl, Aryl oder Aryl-niederes Alkyl, $R^5$ Hydroxy und $R^{41}$ Wasserstoff, niederes Alkyl, Aryl, Aryl-niederes Alkyl oder die Gruppe $-A-R^5$ bedeuten, wobei A und $R^5$ obige Bedeutung besitzen, und, wenn B eine Carbonylgruppe bedeutet, die gestrichelte Linie eine fakultative Bindung bedeutet.

15. Verbindungen der allgemeinen Formel

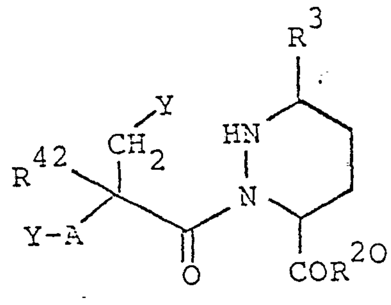

V

worin A gegebenenfalls durch niederes Alkyl substituiertes Methylen, Aethylen oder Propylen, X Halogen, $R^3$ Wasserstoff, niederes Alkyl, Aryl oder Aryl-niederes Alkyl, $R^{20}$ niederes Alkoxy und $R^{42}$ Wasserstoff, niederes Alkyl, Aryl, Aryl-niederes Alkyl oder die Gruppe -A-Y bedeuten, wobei A und Y obige Bedeutung besitzen.

16. Verbindungen der allgemeinen Formel

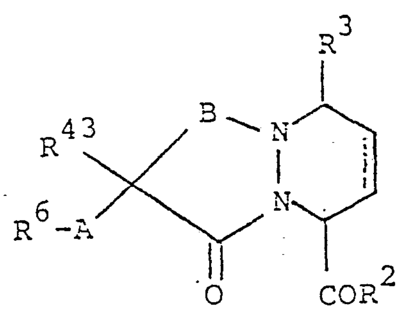

VI

worin A gegebenenfalls durch niederes Alkyl substituiertes Methylen, Aethylen oder Propylen, B eine Carbonyl- oder Methylengruppe, $R^2$ Hydroxy, niederes Alkoxy oder Amino, $R^3$ Wasserstoff, niederes Alkyl, Aryl oder Aryl-niederes Alkyl, $R^6$ niederes Alkylsulfonyloxy oder Arylsulfonyloxy und $R^{43}$ Wasserstoff, niederes Alkyl, Aryl, Aryl-niederes Alkyl oder die Gruppe $-A-R^6$ bedeuten, wobei A und $R^6$ obige Bedeutung besitzen, und, wenn B eine Carbonylgruppe bedeutet, die gestrichelte Linie eine fakultative Bindung bedeutet.

17. Verbindungen der allgemeinen Formel

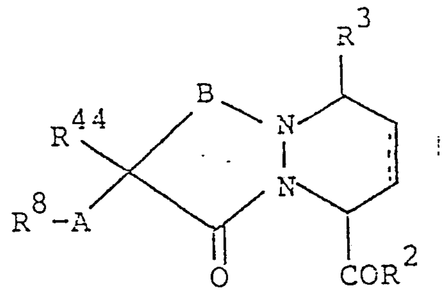

VIII

worin A gegebenenfalls durch niederes Alkyl substituiertes Methylen, Aethylen oder Propylen, B eine Carbonyl- oder Methylengruppe, $R^2$ Hydroxy, niederes Alkoxy oder Amino, $R^3$ Wasserstoff, niederes Alkyl, Aryl oder Aryl-niederes Alkyl, $R^8$ Aryloxycarbonyl und $R^{44}$ Wasserstoff, niederes Alkyl, Aryl, Aryl-niederes Alkyl oder die Gruppe $-A-R^8$ bedeuten, wobei A und $R^8$ obige Bedeutung besitzen, und, wenn B eine Carbonylgruppe bedeutet, die gestrichelte Linie eine fakultative Bindung bedeutet.

18. Verbindungen der allgemeinen Formel

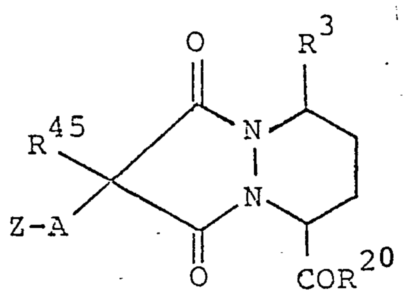

IX

worin A gegebenenfalls durch niederes Alkyl substituiertes Methylen, Aethylen oder Propylen, $R^{20}$ niederes Alkoxy, $R^3$ Wasserstoff, niederes Alkyl, Aryl oder Aryl-niederes Alkyl, Z Benzyloxycarbonyl und $R^{45}$ Wasserstoff, niederes Alkyl, Aryl, Aryl-niederes Alkyl oder die Gruppe -A-Z bedeuten, wobei A und Z obige Bedeutung besitzen.

19. Verbindungen gemäss einem der Ansprüche 1 bis 12 als pharmazeutische Wirkstoffe.

20. Verbindungen gemäss einem der Ansprüche 1 bis 12 als antihypertensive Wirkstoffe.

21. Verfahren zur Herstellung von Verbindungen gemäss einem der Ansprüche 1 bis 12, dadurch gekennzeichnet, dass man

a) zur Herstellung einer Verbindung der allgemeinen Formel I, worin B eine Carbonylgruppe, $R^1$ niederes Alkoxycarbonyl oder niederes Alkanoylthio und $R^2$ niederes Alkoxy bedeuten und in 6,7-Stellung eine Einfachbindung vorhanden ist, eine Verbindung der allgemeinen Formel

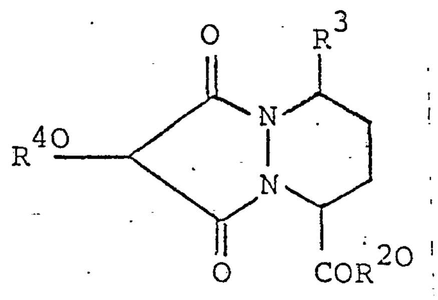

II

worin $R^3$ die in Anspruch 1 angegebene Bedeutung besitzt, $R^{20}$ niederes Alkoxy und $R^{40}$ Wasserstoff, niederes Alkyl, Aryl oder Aryl-niederes Alkyl bedeuten,

mit einer Verbindung der allgemeinen Formel

$$R^{11}\text{-A-X} \qquad \text{III}$$

worin A die in Anspruch 1 angegebene Bedeutung besitzt, $R^{11}$ niederes Alkoxycarbonyl oder niederes Alkanoylthio und X eine Abgangsgruppe bedeuten,

umsetzt, oder

b) zur Herstellung einer Verbindung der allgemeinen Formel I, worin $R^1$ Halogen bedeutet, eine Verbindung der allgemeinen Formel

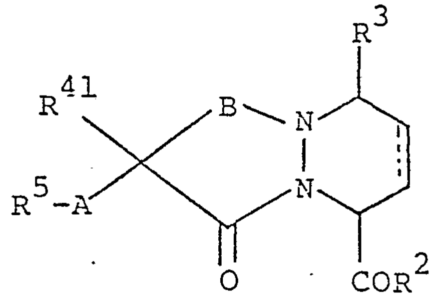

IV

worin A, B, $R^2$, $R^3$ und die gestrichelte Linie die in Anspruch 1 angegebene Bedeutung besitzen, $R^5$ Hydroxy und $R^{41}$ Wasserstoff, niederes Alkyl, Aryl, Aryl-niederes Alkyl oder die Gruppe $-A-R^5$ bedeuten,
entsprechend halogeniert, oder

c) zur Herstellung einer Verbindung der allgemeinen Formel I, worin B eine Methylengruppe, $R^1$ Halogen und $R^2$ niederes Alkoxy bedeuten, eine Verbindung der allgemeinen Formel

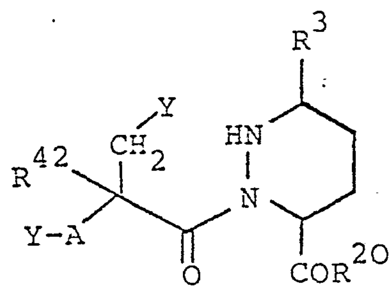

V

worin A, $R^3$ und $R^{20}$ die in Anspruch 1 angegebene Bedeutung besitzen, Y Halogen und $R^{42}$ Wasserstoff, niederes Alkyl, Aryl, Aryl-niederes Alkyl oder die Gruppe -A-Y bedeuten,
cyclisiert, oder

d) zur Herstellung einer Verbindung der allgemeinen Formel I, worin $R^1$ niederes Alkoxycarbonyl bedeutet, in einer entsprechenden Verbindung der allgemeinen Formel I, worin $R^1$ Halogen bedeutet, das Halogenatom in an sich bekannter Weise durch niederes Alkoxycarbonyl ersetzt, oder

e) zur Herstellung einer Verbindung der allgemeinen Formel I, worin $R^1$ niederes Alkanoylthio oder Aryl-niederes Alkylthio bedeutet, eine entsprechende Verbindung der allgemeinen Formel I, worin $R^1$ Halogen bedeutet, oder eine Verbindung der allgemeinen Formel

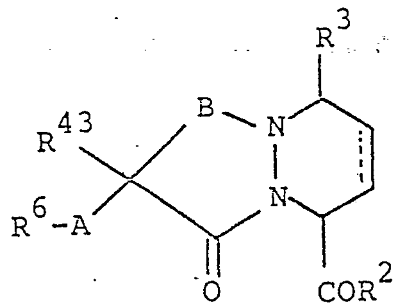

VI

worin A, B, $R^2$, $R^3$ und die gestrichelte Linie die in Anspruch 1 angegebene Bedeutung besitzen, $R^6$ niederes Alkylsulfonyloxy oder Arylsulfonyloxy und $R^{43}$ Wasserstoff, niederes Alkyl, Aryl, Aryl-niederes Alkyl oder die Gruppe $-A-R^6$ bedeuten,

mit einer Verbindung der allgemeinen Formel

$$R^7-SH \qquad VII$$

worin $R^7$ niederes Alkanoyl oder Aryl-niederes Alkyl bedeutet,

umsetzt, oder

f) zur Herstellung einer Verbindung der allgemeinen Formel I, worin $R^1$ Mercapto bedeutet, in einer entsprechenden Verbindung der allgemeinen Formel I, worin $R^1$ niederes Alkanoylthio oder Aryl-niederes Alkylthio bedeutet, die niedere Alkanoyl- oder Aryl-niedere Alkylgruppe abspaltet, oder

g) zur Herstellung einer Verbindung der allgemeinen Formel I, worin B eine Carbonylgruppe, $R^1$ Halogen, Carboxyl, niederes Alkoxycarbonyl oder Hydroxyaminocarbonyl bedeuten und in 6,7-Stellung eine Einfachbindung vorhanden ist,

eine entsprechende Verbindung der allgemeinen Formel I, worin in 6,7-Stellung eine Doppelbindung vorhanden ist, katalytisch hydriert, oder

h) zur Herstellung einer Verbindung der allgemeinen Formel I, worin $R^1$ Hydroxyaminocarbonyl bedeutet, eine entsprechende Verbindung der allgemeinen Formel I, worin $R^1$ niederes Alkoxycarbonyl bedeutet, oder eine Verbindung der allgemeinen Formel

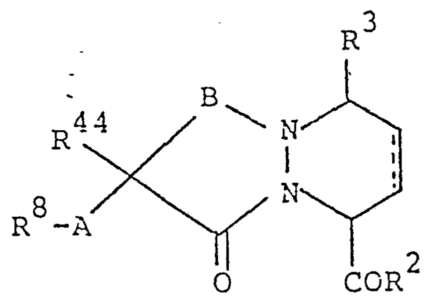

VIII

worin A, B, $R^2$, $R^3$ und die gestrichelte Linie die in Anspruch 1 angegebene Bedeutung besitzen, $R^8$ Aryloxycarbonyl und $R^{44}$ Wasserstoff, niederes Alkyl, Aryl, Aryl-niederes Alkyl oder die Gruppe $-A-R^8$ bedeuten, mit Hydroxylamin umsetzt, oder

i) zur Herstellung einer Verbindung der allgemeinen Formel I, worin $R^1$ niederes Alkoxycarbonyl und/oder $R^2$ niederes Alkoxy bedeuten, eine entsprechende Verbindung der allgemeinen Formel I, worin $R^1$ Carboxy und/oder $R^2$ Hydroxy bedeuten, verestert, oder

j) zur Herstellung einer Verbindung der allgemeinen Formel I, worin $R^2$ Amino bedeutet, eine entsprechende Verbindung der allgemeinen Formel I, worin $R^2$ Hydroxy bedeutet, amidiert, oder

k) zur Herstellung einer Verbindung der allgemeinen Formel I, worin $R^1$ Carboxy und/oder $R^2$ Hydroxy bedeuten, eine entsprechende Verbindung der allgemeinen Formel I, worin $R^1$ niederes Alkoxycarbonyl und/oder $R^2$ niederes

Alkoxy bedeuten, mit einer Säure oder mit einer Base behandelt, oder

l) zur Herstellung einer Verbindung der allgemeinen Formel I, worin B eine Carbonylgruppe, $R^1$ Carboxyl und $R^2$ niederes Alkoxy bedeuten und in 6,7-Stellung eine Einfachbindung vorhanden ist, eine Verbindung der allgemeinen Formel

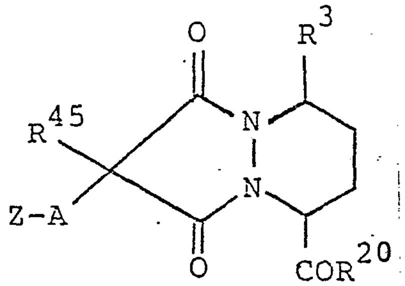

IX

worin A, $R^3$ und $R^{20}$ die in Anspruch 1 angegebene Bedeutung besitzen, Z Benzyloxycarbonyl und $R^{45}$ Wasserstoff, niederes Alkyl, Aryl, Aryl-niederes Alkyl oder die Gruppe -A-Z bedeuten,

debenzyliert, oder

m) zur Herstellung einer Verbindung der allgemeinen Formel I, worin $R^1$ Carboxy bedeutet, eine Verbindung der obigen allgemeinen Formel IV oxydiert, und

n) erwünschtenfalls ein erhaltenes Gemisch von Diastereoisomeren in die entsprechenden Racemate auftrennt, und/oder

o) erwünschtenfalls ein erhaltenes Racemat in die entsprechenden Antipoden spaltet und/oder

p) erwünschtenfalls eine erhaltene Verbindung der allgemeinen Formel I, worin $R^1$ Carboxy und/oder $R^2$ Hydroxy bedeuten, mit einer pharmazeutisch annehmbaren Base in ein Salz überführt.

22. Verfahren gemäss Anspruch 21 zur Herstellung einer Verbindung der in Anspruch 1 definierten allgemeinen Formel I, worin B eine Carbonylgruppe bedeutet, und von Salzen von solchen Verbindungen, worin $R^1$ Carboxy und/oder $R^2$ Hydroxy bedeuten, mit pharmazeutisch annehmbaren Basen, dadurch gekennzeichnet, dass man nach Verfahrensvariante a) unter Verwendung einer Verbindung der allgemeinen Formel II, worin $R^3$ Wasserstoff bedeutet, oder nach Verfahrensvariante b) unter Verwendung einer Verbindung der allgemeinen Formel IV, worin B eine Carbonylgruppe bedeutet, und in 6,7-Stellung eine Doppelbindung vorhanden ist, oder nach Verfahrensvariante d) unter Verwendung einer entsprechenden Verbindung der allgemeinen Formel I, worin in 6,7-Stellung eine Doppelbindung vorhanden ist, oder nach Verfahrensvariante e) unter Verwendung einer Verbindung der allgemeinen Formel I, worin $R^1$ Halogen bedeutet, oder nach Verfahrensvariante f) oder g), oder nach Verfahrensvariante h) unter Verwendung einer entsprechenden Verbindung der allgemeinen Formel I, worin $R^1$ niederes Alkoxycarbonyl bedeutet, oder nach Verfahrensvariante i), j) oder k), oder nach Verfahrensvariante l) unter Verwendung einer Verbindung der allgemeinen Formel IX, worin $R^3$ Wasserstoff bedeutet, oder nach Verfahrensvariante n), o) und/oder p) verfährt.

23. Arzneimittel, enthaltend eine Verbindung gemäss einem der Ansprüche 1 bis 12.

24. Antihypertensive Mittel, enthaltend eine Verbindung gemäss einem der Ansprüche 1 bis 12.

***

Patentansprüche **für Oesterreich**

1. Verfahren zur Herstellung von Pyrazolopyridazin-Derivaten der allgemeinen Formel

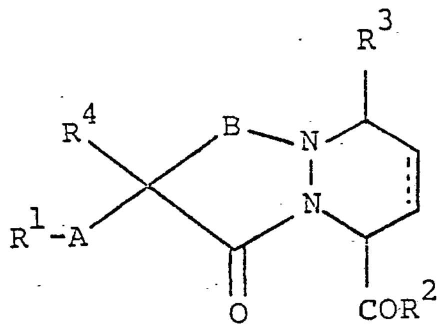

I

worin A gegebenenfalls durch niederes Alkyl substituiertes Methylen, Aethylen oder Propylen, B eine Carbonyl- oder Methylengruppe, $R^1$ Halogen, Carboxyl, niederes Alkoxycarbonyl, Hydroxyaminocarbonyl, Mercapto, niederes Alkanoylthio oder Aryl-niederes Alkylthio, $R^2$ Hydroxy, niederes Alkoxy oder Amino und $R^3$ Wasserstoff, niederes Alkyl, Aryl oder Aryl-niederes Alkyl bedeuten, $R^4$ Wasserstoff, niederes Alkyl, Aryl, Aryl-niederes Alkyl oder die Gruppe $-A-R^1$ bedeutet, wobei A und $R^1$ obige Bedeutung besitzen, und die gestrichelte Linie eine fakultative Bindung bedeutet, wenn B eine Carbonylgruppe bedeutet,

und von Salzen von Verbindungen der allgemeinen Formel I, worin $R^1$ Carboxy und/oder $R^2$ Hydroxy bedeuten, mit pharmazeutisch annehmbaren Basen, dadurch gekennzeichnet, dass man

a) zur Herstellung einer Verbindung der allgemeinen Formel I, worin B eine Carbonylgruppe, $R^1$ niederes Alkoxycarbonyl oder niederes Alkanoylthio und $R^2$ niederes Alkoxy bedeuten und in 6,7-Stellung eine Einfachbindung vorhanden ist, eine Verbindung der allgemeinen Formel

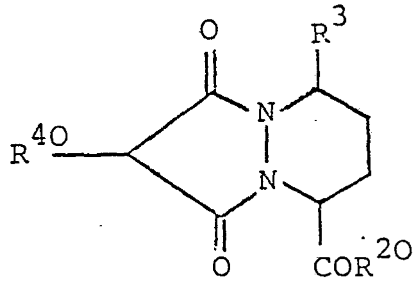

II

worin $R^3$ obige Bedeutung besitzt, $R^{20}$ niederes Alkoxy und $R^{40}$ Wasserstoff, niederes Alkyl, Aryl oder Aryl-niederes Alkyl bedeuten,

mit einer Verbindung der allgemeinen Formel

$$R^{11}\text{-A-X} \qquad \text{III}$$

worin A obige Bedeutung besitzt, $R^{11}$ niederes Alkoxycarbonyl oder niederes Alkanoylthio und X eine Abgangsgruppe bedeuten,

umsetzt, oder

b) zur Herstellung einer Verbindung der allgemeinen Formel I, worin $R^1$ Halogen bedeutet, eine Verbindung der allgemeinen Formel

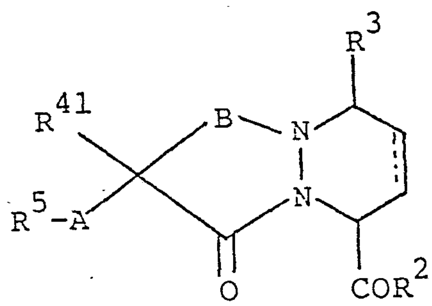

IV

worin A, B, $R^2$, $R^3$ und die gestrichelte Linie obige Bedeutung besitzen, $R^5$ Hydroxy und $R^{41}$ Wasserstoff, niederes Alkyl, Aryl, Aryl-niederes Alkyl oder die Gruppe $-A-R^5$ bedeuten,

entsprechend halogeniert, oder

c) zur Herstellung einer Verbindung der allgemeinen Formel I, worin B eine Methylengruppe, $R^1$ Halogen und $R^2$

niederes Alkoxy bedeuten, eine Verbindung der allgemeinen Formel

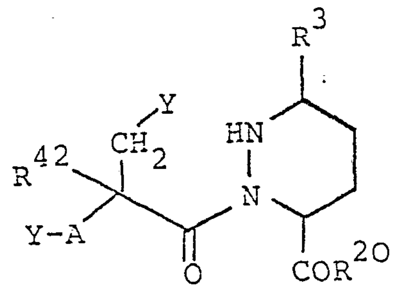

V

worin A, $R^3$ und $R^{20}$ obige Bedeutung besitzen, Y Halogen und $R^{42}$ Wasserstoff, niederes Alkyl, Aryl, Aryl-niederes Alkyl oder die Gruppe -A-Y bedeuten, cyclisiert, oder

d) zur Herstellung einer Verbindung der allgemeinen Formel I, worin $R^1$ niederes Alkoxycarbonyl bedeutet, in einer entsprechenden Verbindung der allgemeinen Formel I, worin $R^1$ Halogen bedeutet, das Halogenatom in an sich bekannter Weise durch niederes Alkoxycarbonyl ersetzt, oder

e) zur Herstellung einer Verbindung der allgemeinen Formel I, worin $R^1$ niederes Alkanoylthio oder Aryl-niederes Alkylthio bedeutet, eine entsprechende Verbindung der allgemeinen Formel I, worin $R^1$ Halogen bedeutet, oder eine Verbindung der allgemeinen Formel

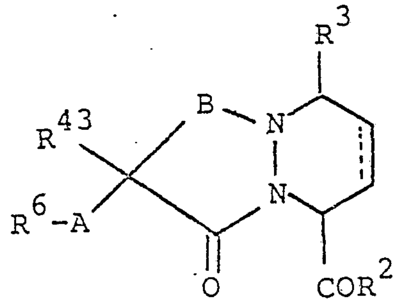

VI

worin A, B, $R^2$, $R^3$ und die gestrichelte Linie obige Bedeutung besitzen, $R^6$ niederes Alkylsulfonyloxy oder Arylsulfonyloxy und $R^{43}$ Wasserstoff, niederes Alkyl, Aryl, Aryl-niederes Alkyl oder die Gruppe $-A-R^6$ be-

deuten,

mit einer Verbindung der allgemeinen Formel

$R^7$-SH VII

worin $R^7$ niederes Alkanoyl oder Aryl-niederes Alkyl bedeutet,

umsetzt, oder

f) zur Herstellung einer Verbindung der allgemeinen Formel I, worin $R^1$ Mercapto bedeutet, in einer entsprechenden Verbindung der allgemeinen Formel I, worin $R^1$ niederes Alkanoylthio oder Aryl-niederes Alkylthio bedeutet, die niedere Alkanoyl- oder Aryl-niedere Alkylgruppe abspaltet, oder

g) zur Herstellung einer Verbindung der allgemeinen Formel I, worin B eine Carbonylgruppe, $R^1$ Halogen, Carboxyl, niederes Alkoxycarbonyl oder Hydroxyaminocarbonyl bedeuten und in 6,7-Stellung eine Einfachbindung vorhanden ist, eine entsprechende Verbindung der allgemeinen Formel I, worin in 6,7-Stellung eine Doppelbindung vorhanden ist, katalytisch hydriert, oder

h) zur Herstellung einer Verbindung der allgemeinen Formel I, worin $R^1$ Hydroxyaminocarbonyl bedeutet, eine entsprechende Verbindung der allgemeinen Formel I, worin $R^1$ niederes Alkoxycarbonyl bedeutet, oder eine Verbindung der allgemeinen Formel

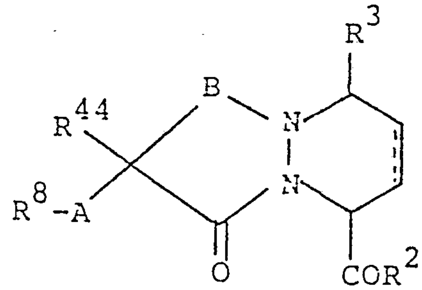

VIII

worin A, B, $R^2$, $R^3$ und die gestrichelte Linie obige Bedeutung besitzen, $R^8$ Aryloxycarbonyl und $R^{44}$ Wasserstoff, niederes Alkyl, Aryl, Aryl-niederes Alkyl oder die Gruppe $-A-R^8$ bedeuten,
mit Hydroxylamin umsetzt, oder

i) zur Herstellung einer Verbindung der allgemeinen Formel I, worin $R^1$ niederes Alkoxycarbonyl und/oder $R^2$ niederes Alkoxy bedeuten, eine entsprechende Verbindung der allgemeinen Formel I, worin $R^1$ Carboxy und/oder $R^2$ Hydroxy bedeuten, verestert, oder

j) zur Herstellung einer Verbindung der allgemeinen Formel I, worin $R^2$ Amino bedeutet, eine entsprechende Verbindung der allgemeinen Formel I, worin $R^2$ Hydroxy bedeutet, amidiert, oder

k) zur Herstellung einer Verbindung der allgemeinen Formel I, worin $R^1$ Carboxy und/oder $R^2$ Hydroxy bedeuten, eine entsprechende Verbindung der allgemeinen Formel I, worin $R^1$ niederes Alkoxycarbonyl und/oder $R^2$ niederes Alkoxy bedeuten, mit einer Säure oder mit einer Base behandelt, oder

l) zur Herstellung einer Verbindung der allgemeinen Formel I, worin B eine Carbonylgruppe, $R^1$ Carboxyl und $R^2$ niederes Alkoxy bedeuten und in 6,7-Stellung eine Einfachbindung vorhanden ist, eine Verbindung der allgemeinen Formel

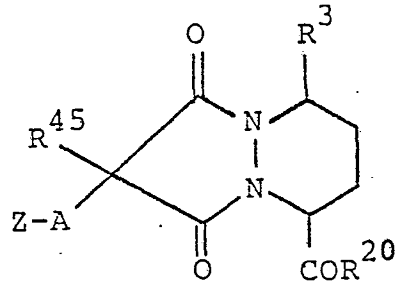

IX

worin A, $R^3$ und $R^{20}$ obige Bedeutung besitzen, Z Benzyloxycarbonyl und $R^{45}$ Wasserstoff, niederes Alkyl, Aryl, Aryl-niederes Alkyl oder die Gruppe -A-Z bedeuten,

debenzyliert, oder

m) zur Herstellung einer Verbindung der allgemeinen Formel I, worin $R^1$ Carboxy bedeutet, eine Verbindung der obigen allgemeinen Formel IV oxydiert, und

n) erwünschtenfalls ein erhaltenes Gemisch von Diastereoisomeren in die entsprechenden Racemate auftrennt, und/oder

o) erwünschtenfalls ein erhaltenes Racemat in die entsprechenden Antipoden spaltet und/oder

p) erwünschtenfalls eine erhaltene Verbindung der allgemeinen Formel I, worin $R^1$ Carboxy und/oder $R^2$ Hydroxy bedeuten, mit einer pharmazeutisch annehmbaren Base in ein Salz überführt.

2. Verfahren gemäss Anspruch 1 zur Herstellung einer Verbindung der in Anspruch 1 definierten allgemeinen Formel I, worin B eine Carbonylgruppe bedeutet, und von Salzen von solchen Verbindungen, worin $R^1$ Carboxy und/oder $R^2$ Hydroxy bedeuten, mit pharmazeutisch annehmbaren Basen, dadurch gekennzeichnet, dass man nach Verfahrensvariante a) unter Verwendung einer Verbindung der allgemeinen Formel II, worin $R^3$ Wasserstoff bedeutet, oder nach Verfahrensvariante b) unter Verwendung einer Verbindung der allgemeinen Formel IV, worin B eine Carbonylgruppe bedeutet, und in 6,7-Stellung eine Doppelbindung vorhanden ist, oder nach Verfahrensvariante d) unter Verwendung einer entsprechenden Verbindung der allgemeinen Formel I, worin in 6,7-Stellung eine Doppelbindung vorhanden ist, oder nach Verfahrensvariante e) unter Verwendung einer Verbindung der allgemeinen Formel I, worin $R^1$ Halogen bedeutet, oder nach Verfahrensvariante f) oder g), oder nach Verfahrensvariante h) unter Verwendung einer entsprechenden Verbindung der allgemeinen Formel I, worin $R^1$ niederes Alkoxycarbonyl bedeutet, oder nach Verfahrensvariante i), j) oder k), oder nach Verfahrensvariante l) unter Verwendung einer Verbindung der allgemeinen Formel IX, worin $R^3$ Wasserstoff bedeutet, oder nach Verfahrensvariante n), o) und/oder p) verfährt.

3. Verfahren gemäss Anspruch 1 oder 2, dadurch gekennzeichnet, dass man eine Verbindung der in Anspruch 1 definierten allgemeinen Formel I herstellt, worin B eine Carbonylgruppe bedeutet.

4. Verfahren gemäss einem der Ansprüche 1 bis 3, dadurch gekennzeichnet, dass man eine Verbindung der in Anspruch 1 definierten allgemeinen Formel I herstellt, worin $R^1$ Mercapto bedeutet.

5. Verfahren gemäss einem der Ansprüche 1 bis 4, dadurch gekennzeichnet, dass man eine Verbindung der in Anspruch 1 definierten allgemeinen Formel I herstellt, worin $R^2$ Hydroxy bedeutet.

6. Verfahren gemäss einem der Ansprüche 1 bis 5, dadurch gekennzeichnet, dass man eine Verbindung der in Anspruch 1 definierten allgemeinen Formel I herstellt, worin $R^3$ Wasserstoff bedeutet.

7. Verfahren gemäss einem der Ansprüche 1 bis 6, dadurch gekennzeichnet, dass man eine Verbindung der in Anspruch 1 definierten allgemeinen Formel I herstellt, worin $R^4$ Wasserstoff, niederes Alkyl, vorzugsweise Methyl oder Aethyl, oder Aryl-niederes Alkyl, vorzugsweise Benzyl, bedeutet.

8. Verfahren gemäss einem der Ansprüche 1 bis 7, dadurch gekennzeichnet, dass man eine Verbindung der in Anspruch 1 definierten allgemeinen Formel I herstellt,

worin B eine Carbonylgruppe, $R^1$ Mercapto, $R^2$ Hydroxy, $R^3$ Wasserstoff und $R^4$ Wasserstoff, Methyl, Aethyl oder Benzyl bedeuten.

9. Verfahren gemäss Anspruch 1, dadurch gekennzeichnet, dass man 2,3,5,8-Tetrahydro-2-(2-mercaptoäthyl)-2-methyl-1,3-dioxo-1H-pyrazolo[1,2-a]pyridazin-5-carbonsäure herstellt.

Europäisches Patentamt

**EUROPÄISCHER RECHERCHENBERICHT**

0042100 Nummer der Anmeldung

EP 81104239.9

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| A | DE - A - 2 051 800 (GRUPPE LEPETIT)<br>* Seitén 1-3 *<br>---- | 1 | C 07 D 487/04<br>C 07 D 237/04<br>A 61 K 31/50//<br>(C 07 D 487/04<br>C 07 D 237/00<br>C 07 D 231/00) |

RECHERCHIERTE SACHGEBIETE (Int. Cl.³)

C 07 D 487/00
C 07 D 237/00
A 61 K 31/00

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 08-09-1981 | BRUS |

EPA form 1503.1 06.78